## Europäisches Patentamt

(19) **European Patent Office**

**Office européen des brevets**

(11) Veröffentlichungsnummer : **0 312 698 B1**

(12) **EUROPÄISCHE PATENTSCHRIFT**

(45) Veröffentlichungstag der Patentschrift :
03.07.91 Patentblatt 91/27

(51) Int. Cl.⁵ : **A61C 8/00**

(21) Anmeldenummer : **88110095.2**

(22) Anmeldetag : **04.11.85**

(54) Enossales Implantat zur Befestigung von festsitzendem oder abnehmbarem Zahnersatz.

(30) Priorität : 09.11.84 DE 3440952
26.04.85 DE 3515154
10.09.85 DE 3532125

(43) Veröffentlichungstag der Anmeldung :
26.04.89 Patentblatt 89/17

(45) Bekanntmachung des Hinweises auf die
Patenterteilung :
03.07.91 Patentblatt 91/27

(84) Benannte Vertragsstaaten :
AT BE CH DE FR GB IT LI LU NL SE

(56) Entgegenhaltungen :
EP-A- 0 083 028
DE-A- 3 300 764
FR-A- 2 050 612

(61) Veröffentlichungsnummer der früheren
Anmeldung nach Art. 76 EPü : 0180247

(73) Patentinhaber : IMPLANTO-LOcK Gesellschaft
mit beschränkter Haftung für
Implantatforschung und Entwicklung
Am Husarendenkmal 13
W-2000 Hamburg 70 (DE)

(72) Erfinder : Koch, Werner Lutz
Lönsweg 7
W-3073 Liebenau (DE)

(74) Vertreter : Richter, Werdermann & Gerbaulet
Neuer Wall 10
W-2000 Hamburg 36 (DE)

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein enossales Implantat zur Befestigung von festsitzendem oder abnehmbarem Zahnersatz, bestehend aus zwei miteinander verbindbaren Teilen, von denen das eine Teil als in den Kieferknochen einbringbarer und sich in diesem kraftschlüssig verankernder Primärzylinder mit einer mittigen Längsbohrung und das andere Teil als Sekundärzylinder ausgebildet ist, der einen in die Längsbohrung des Primärzylinders einbringbaren und in diesem gehaltenen Implantatpfosten zur Aufnahme des Zahnersatzes aufweist, und aus einem an dem Implantatpfosten vorgesehenen Implantataufsatz, wobei der Implantatpfosten an seinem freien oberen Ende für den Anschluß des Zahnersatzes ausgebildet ist und von einem die im Bereich des Zahnersatzes oder im Munde auftretenden Horizontalund/oder Vertikal- und/oder Torsions-Kräfte und Schwingungen in den Sekundärzylinder und von dort in den Primärzylinder umleitenden Kraftleitungssystem aus mehreren, sich aneinanderreihenden Bereichen mit unterschiedlichen elastischen Eigenschaften umgeben und in dem Primärzylinder fest oder lösbar gehalten ist.

Durch die EP-A-0 083 028 ist ein Verbindungselement für einen Implantatkörper und eine Suprastruktur mit den Merkmalen des Oberbegriffes des Anspruches 1 bekannt. Bei diesem Verbindungselement besteht der Implantatpfosten aus einem Werkstoff mit einem hohen Elastizitätsmodul und weist eine Verdickung auf, die aus dem gleichen oder einem ähnlichen Werkstoff wie der Implantatpfosten besteht. Außerdem weist das Verbindungselement eine den Implantatpfosten zusammen mit seiner Verdickung umfassende Umhüllung auf, die in ihrer äußeren Form der Bohrung des Implantats entspricht und aus einem Werkstoff besteht, das den gleichen oder einen niedrigeren Elastizitätsmodul aufweist als der Werkstoff, aus dem der Implantatpfosten besteht.

Da die Verdickung des Implantatpfostens aus dem gleichen Werkstoff wie dieser besteht, z.B. aus Metall oder Keramik, und direkt an der Wand der Bohrung des Implantats anliegt, so wie etwa in halber Höhe der Implantatbohrung an dem Implantatpfosten vorgesehen ist, ergibt sich beim Angreifen von Horizontalkräften auf den am freien oberen Ende des Implantatpfostens befestigten Zahnersatzes ein Verschwenken des Implantatpfostens um die Lagerpunkte der Verdickung an der Wandfläche der Implantatbohrung, so daß der Hebeldrehpunkt etwa in der Mitte der Verdickung zu liegen kommt, wenn diese eine kugelförmige Gestalt aufweist. Dadurch ergibt sich zwangsläufig, daß der Implantatpfosten als zweiarmiger Hebel wirkt, so daß ein Ableiten auftretender Kräfte über die Verdickung in den Implantatkörper erfolgt. Da das untere Ende des Implantatpfostens dann frei verschwenkbar ist, ist ein Ableiten auftretender Kräfte in den bodenseitigen Bereich des Implantatkörpers nicht möglich.

Des weiteres ist bei dem bekannten Implantat eine den Implantatpfosten mit seiner Verdickung umfassende Umhüllung vorgesehen, die aus einem Werkstoff mit einem niederen Elastizitätsmodul als der Implantatpfosten und seine Verdickung besteht. Als Werkstoff für diese Umhüllung werden geeignete Kunststoffe, wie z.B. Polyäthylen, eingesetzt. Dadurch weist die Umhüllung eine Elastizität auf, und zwar sowohl in den Bereichen oberhalb der Verdickung an dem Implantatpfosten als auch unterhalb dieser Verdickung. Dadurch ist der Implantatpfosten bodenseitig nicht fest in einem Umhüllungsabschnitt aus einem unelastischen Werkstoff eingebunden, so daß auch in diesem Fall ein Ableiten auftretender Spannungskräfte im bodenseitigen Bereich des Implantatkörpers nicht möglich ist, da auftretende Spannungskräfte von der elastischen Umhüllung aufgenommen werden, es kommt somit zu einer Druckabsorption durch den elastischen Werkstoff der Umhüllung Außerdem behält der Implantatpfosten seine Wirkung als zweiarmiger Hebel bei, da die Umhüllung in den Bereichen oberhalb und unterhalb der Verdickung aus einem elastischen Werkstoff besteht. Wesentlich ist jedoch bei diesem bekannten Implantat, daß der Implantatpfosten im bodenseitigen Bereich in der Implantatbohrung federnd gelagert ist.

Des weiteren ist das bodenseitige Ende des Implantatpfostens in einer Führungskappe angeordnet, die am Boden der Implantatbohrung angeordnet ist. Diese Führungskappe besteht aus einem Werkstoff mit einem höheren Elastizitätsmodul als der übrige Teil der Umhüllung, so daß letztlich diese Führungskappe eine Elastizität besitzt, die zwar geringer ist als die Elastizität der übrigen Umhüllung, jedoch besteht die Führungskappe nicht aus einem unelastischen Werkstoff, aus dem der Implantatpfosten mit seiner Verdickung besteht. Der Implantatpfosten behält seine Wirkung als zweiarmiger Hebel immer bei, denn bei an der Implantatkrone angreifenden Horizontalkräften verteilen sich diese zu gleichen Teilen auf die Nachbarpfeiler, wobei der für das Implantat verbleibende Anteil in Höhe der Verdickung und nicht im bodenseitigen Bereich des Implantats angreift.

Auch wenn die Umhüllung aus Werkstoffen mit unterschiedlichem Elastizitätsmodul besteht oder wenn die Umhüllung abschnittsweise entfällt, wird ein Ableiten auftretender Spannungskräfte in den Boden des Implantats nicht erreicht, da der Implantatpfosten bodenseitig elastisch gelagert ist.

Nachteilig ist somit bei diesem Implantat, daß der Hebeldrehpunkt des Implantatpfostens etwa in der Mitte des Implantatkörpers liegt, so daß ein Auslockern des Implantats nicht mit Sicherheit vermeidbar ist. Außerdem ist ein einwandfreies Ableiten auftretender Spannungskräfte in den Außenbereich des Implantatikörpers nicht gewährleistet, so daß es zu einer Beschädigung und bei hoher horizontaler Druckbelastung des Zahnersatzes zu einem Bruch des Implantatkörpers kommen kann, insbesondere dann, wenn der Implantatkörper aus einem Keramik-Werkstoff besteht.

Die Erfindung löst die Aufgabe, ein aus einem Primärzylinder und einem in diesem gehaltenen Sekundärzylinder bestehendes enossales Implantat zu schaffen, mit dem ein form- und kraftschlüssiger Verbund zum Knochen hin erreicht wird und bei dem eine belastungsfreie Stabilisierung des Primärzylinders sichergestellt ist und das Implantat keinerlei plastischen Verformungen unterliegt und eine wartungsfreie Mechanik aufweist. Des weiteren soll eine feste Verbindung des Implantatpfostens des Sekundärzylinders in der Längsbohrung des Primärzylinders geschaffen werden, ohne daß die schwingende Eigenschaft des Implantatpfostens beeinträchtigt wird, wobei die im Munde auftretenden Horizontal- und/oder Vertikal- und/oder Torsionskräfte in den Boden des Primärzylinders abgeleitet werden sollen und das Einführen des Implantatpfostens in die Längsbohrung des Primärzylinders erleichtert werden soll.

Diese Aufgabe wird bei einem gattungsgemäßen enossalen Implantat erfindungsgemäß durch die im Anspruch 1 gekennzeichneten Merkmale gelöst.

Hiernach ist das Implantat in der Weise ausgebildet, daß der Implantatpfosten von einem die im Bereich des Zahnersatzes durch auf diesen einwirkende Kaumuskelkraft auftretenden Schwingungen bei gleichzeitiger Verlagerung des Hebeldrehpunktes des Implantatpfostens in den unteren Bereich des Sekundärzylinders von dort in den Primärzylinder umleitenden Kraftleitungssystem umgeben ist, das aus einem den Implantatpfosten im Bereich des Implantataufsatzes umgebenden Modulelement aus einem stark elastischen Werkstoff besteht.

Nach einer weiteren Ausführungsform der Erfindung besteht das Kraftleitungssystem aus einem den Implantatpfosten im Bereich des Implantataufsatzes umgebenden Modulelement aus einem stark elastischen Werkstoff, aus einem sich von der Längsbohrungseintrittsöffnung erstreckenden, einen Luftspalt bildenden, modulelementfreien Abschnitt und aus einem in einem Abstand von der Längsbohrungseintrittsöffnung angeordneten und sich an den modulelementfreien Abschnitt anschließenden Führungsrohr aus einem elastischen Werkstoff, das in dem Primärzylinder mittels einer Klebeverbindung gehalten ist.

Der mit diesen Ausgestaltungen erreichte Vorteil besteht in folgendem :
Die auf den Implantatpfosten mittels einer Fügeverbindung, z.B. kraftschlüssigen Verbindung mit anaeroben Kunststoffen, aufgesetzten Modulelemente bilden ein Kraftleitungssystem mit unterschiedlichen elastischen Eigenschaften. Die Modulelemente des Kraftleitungssystems sind dabei so angeordnet, daß das Modulelement im Bereich des Implantataufsatzes die größte Elastizität aufweist, wohingegen der Implantatpfosten starr ausgebildet und mit seinem bodenseitigen Ende fest mit dem Primärzylinder verbunden ist. Bei der Einwirkung von Kaumuskelkräften, z.B. horizontal einwirkenden Kräften, auf den Zahnersatz wird der Drehpunkt des als Hebel wirkenden Implantatpfostens in den unteren Bereich des Primärzylinders bzw. des Implantatkörpers verlegt.

Der in dem Primärzylinder angeordnete stabförmige Implantatpfosten besteht aus einem spröden Werkstoff wie z.B. chirurgischem Stahl, und bildet einen Hebel, dessen Drehpunkt aufgrund des speziell ausgebildeten Kraftleitungssystems in das untere Drittel des Primärzylinders verlagert wird. Dadurch, daß der Implantatpfosten von Modulelementen unterschiedlicher Elastizität umgeben ist, werden auftretende Schwingungen am Implantatpfosten bei z.B. senkrecht zur Implantatachse einwirkenden Kaukräften abgefangen, vom Kraftleitungssystem aufgenommen und in den unteren Bereich des Primärzylinders umgeleitet. Die Modulelemente fangen somit die Kräfte ab bzw. leiten diese in den Bereich des Drehpunktes um, also in die Tiefe des Implantats bzw. des Primärzylinders, ohne sich dabei zu deformieren oder plastisch zu verformen. Aufgrund der verwendeten Modulelemente mit unterschiedlichen elastischen Eigenschaften findet bei Krafteinwirkung ein sogenannter Kraftabbau statt, wobei die verbleibenden Kräfte über den Implantatpfosten aus biegefestem Werkstoff zum Drehpunkt im Bereich des Lastarmes des Implantatpfostens umgeleitet werden.

Es ist somit ein aus einem Primärzylinder und einem in diesem gehaltenen Sekundärzylinder bestehendes enossales Implantat mit einem Kraftleitungssystem geschaffen, mit dem eine Umleitung des Kraftflusses vom Krafteinleitungspunkt im Bereich des Zahnersatzes über das Kraftleitungssystem innerhalb des Sekundärzylinders, weiter über die ebenfalls kraftumleitende Führungshülse und über den Primärzylinder in das knöchernde Implantatlager erfolgt, so daß neben einer Reduzierung der Belastungsspitzen und neben einer Vermeidung einer Überbelastung an der Austrittsstelle des Implantats aus dem Knochen der Drehpunkt des Implantatpfostens in den unteren Bereich des Implantats verlegt wird.

Besonders vorteilhaft ist die Ausgestaltung, nach der auf dem Implantatpfosten des Sekundärzylinders ein oberes Modulelement aus einem stark elastischen Werkstoff durch eine Klebeverbindung mit dem Implantatpfosten verbunden angeordnet ist, während der Primärzylinder im Innenraum seiner Längsbohrung in einem Abstand von der Längsbohrungseintrittsöffnung unter Ausbildung eines modulelementfreien, bei eingesetztem Sekundärzylinder einen Luftspalt bildenden Abschnitt ein Führungsrohr aufweist, das in dem Primärzylinder mittels einer Klebeverbindung gehalten ist, wobei auf dem Modulelement ein Implantataufsatz mit einer mit der Längsbohrung des Primärzylinders fluchtenden mittigen Durchbohrung angeordnet ist, der auf der Oberfläche des Primärzylinders gleitend gehalten ist, während der Implantatpfosten des Sekundärzylinders in dem Führungsrohr durch einen an dem Implantatpfosten bei eingesetztem Sekundärzylinder im Bereich des Führungsrohres angeordneten Thermoverschluß gehalten ist, wobei jedoch auch eine unlösbare Befestigung des Implantatpfostens in dem Sekundärzylinder möglich ist. Mit einem derart ausgebildeten Implantat wird ein form- und/oder kraftschlüssiger Verbund von Knochen und Implantat erreicht, wobei dieser Verbund noch durch die Außenbeschichtung des Primärzylinders mit einer Hydroxylapatitkeramik verbessert wird. Hinzu kommt, daß der Primärzylinder belastungsfrei stabilisiert ist und der Sekundärzylinder montagefertig nur aus einem einzigen Teil besteht. Eine Zusammensetzung mehrerer Einzelteile des Sekundärzylinders im Munde des Patienten entfällt, so daß eine einfache und schnelle Handhabung durch den Implanteur gegeben ist. Ferner besteht eine optimale Spaltfreiheit durch die Konstruktion, die eine konstante Zugspannung des Sekundärzylinders gegen den Primärzylinder gewährleistet, und zwar durch die bodenseitige Befestigung des Implantatpfostens in dem Sekundärzylinder, ferner durch die Schaffung von Gleitzonen und durch die Einbringung des Sekundärzylinders in den Primärzylinder unter definiertem Druck. Es erfolgt ferner eine Imitation des Parodontiums durch das Gleiten. Eine absolut wartungs- und verschleißfreie Mechanik liegt vor, da das Implantat nur noch elastisch verformbare Teile aufweist, die keinerlei plastischer Verformung unterliegen, so daß keine plastisch verformbaren Teile mehr ausgetauscht werden müssen. Es entfällt ein großer Teil der Nachsorge durch wartungsfreie Mechanik des Implantats. Dadurch erfolgt eine Gewährleistung der Spaltfreiheit und eine beachtliche Zeitersparnis für den Implanteur. Die Energieflüsse in dem Implantat sind kontrollierbar, da plastisch verformbare Teile vermieden werden, wodurch die Verwendung des bioaktiv beschichteten körperfreundlichen Werkstoffs Aluminiumoxidkeramik gefördert wird, wodurch eine Bruchgefahr ausgeschlossen ist. Dadurch, daß der Implantatpfosten bodenseitig in der Führungshülse des Primärzylinders nach dem Einsetzen des Sekundärzylinders verankert ist, versetzen dynamische, an dem Implantatpfosten ansetzende Vertikal-, Horizontal- und Torsionskräfte den Implantatpfosten in Schwingungen, die in Wärme abgebaut werden, die in das Implantatinnere abgegeben wird. Nicht in Wärme umgesetzte, mengenmäßig geringe mechanische Energien werden im Verschlußpunkt bzw. im Lagerbereich über den Primärzylinder an den Knochen abgegeben. Der Verschlußpunkt ist optimal in der Mitte der Vertikalachse des Primärzylinders plaziert. Die Schwingungsamplituden sind dabei insgesamt so ausgelegt, daß der Primärzylinder mechanisch nicht beansprucht wird. Der abgewinkelte umlaufende Rand des oberen Modulelementes dient zur Kompensation der Implantatpfostenstauchung bei auftretenden Vertikalkräften.

Die Imitation des Parodontiums erfolgt durch kontrollierte Gleitverschiebung des Implantataufsatzes auf dem Primärzylinder, und zwar gedämpft durch das dauerelastische obere Modulelement. Durch die Einbringung des Sekundärzylinders in den Primärzylinder unter definiertem Druck ist Spaltfreiheit durch Chemisorption im Bereich der Gleitzonen sichergestellt. Der Luftspalt oberhalb des Führungsrohres kann durch ein ein- oder aufgesetztes weiteres Modulelement aus dauerelastischem Material auch ausgefüllt sein, das die Spaltdichtigkeit dann gewährleistet. Auch dann, wenn der Implantatpfosten des Sekundärzylinders in seinem unteren Bereich mit dem Führungsrohr mittels einer Klebeverbindung fest verbunden ist, wird durch die feste Verbindung des Implantatpfostens mit der Führungshülse des Primärzylinders erreicht, daß an dem Implantatpfosten ansetzende Vertikal-, Horizontal- und Torsionskräfte den Implantatpfosten in Schwingungen versetzen, die in Wärme abgebaut werden, die in das Implantatinnere abgegeben wird, während nicht in Wärme umgesetzte mengenmäßig geringe mechanische Energien im Verklebungspunkt bzw. im Lagerbereich über den Primärzylinder an den Knochen abgegeben werden.

Es hat sich gezeigt, daß die Keramikoberteile, wie Schleimhautmanschette, bereits bei geringen Kräften von z.B. 5 Kp zerbrechen können, was darauf zurückzuführen ist, daß die sphärische Fläche des Primärzylinders sich bei einem höheren Kraftaufwand keilförmig in das Keramikoberteil schiebt, so daß durch die dabei auftretende Keilwirkung es zu einem Bruch des Keramikoberteils kommen kann.

Auf diese sphärischen Flächen kann jedoch nicht verzichtet werden, weil gerade der "Rotationseffekt" des Implantates seine universelle Verwendung sicherstellt. Hinzu kommt, daß das Schwingungsverhalten des zentralen Implantatpfostens das eines freischwingenden Stabes ist und der Implantatpfosten auch noch bei 250% der üblicherweise im Munde vorkommenden Horizontalkräfte im Implantat frei schwingt, also seine Dämpfungswirkung voll entfalten kann. Dabei wurde festgestellt, daß die Abdichtung des Keramikoberteils gegen das Keramikunterteil und die Gleiteigenschaften (Reibung) durch höhere Anpressung des Oberteils gegen das Unterteil nicht verändert wird und es hierzu lediglich einer geringfügigen Anpressung bedarf, um die erforderliche Abdichtung und das Gleiten sicherzustellen. Es war daher erforderlich, die auf den Implantataufsatz bzw. den Schwingkopf des Implantates einwirkenden Vertikalkräfte in dem Implantatboden und nicht auf dem Keramikoberteil abzufangen.

Aufgrund der im Anspruch 13 angegebenen Ausgestaltung, wonach der Implantatpfosten auch unter Weglassung des Führungsrohres mittels einer Schraubverbindung oder einer anderen geeigneten, gleichwertigen Verbindung mit einem im Innenraum des Primärzylinders mittels einer Klebeverbindung gehaltenen, den gesamten Raum des bisherigen Führungsrohres einschließlich des darunter befindlichen Hohlraumes zwischen dem bodenseitigen Ende des sonst vorgesehenen Führungsrohres und dem Primärzylinderboden ausfüllenden Formkörper mit einer oberen Bohrung für die Aufnahme des Implantatpfostens verbunden ist, werden auf den Schwingkopf des Implantates senkrecht aufgebrachte Kräfte unmittelbar auf den Boden des Implantats verlagert, wobei der Boden des Primärzylinders aus Keramik besteht und werden hier wirksam. Das Keramikoberteil ist entsprechend druckentlastet. Hier können sich nur solche Kräfte auswirken, die aus der Stauchung des Implantatpfostens bei Kraftaufbringung auf den Schwing- bzw. Montagekopf resultieren. Der Implantatpfosten verringert dabei seine Länge jedoch nur unerheblich, z.B. bei Kraftaufbringung von 40 Kp um 41 μ. Eine solche Stauchung wird durch die Elastizität des oberen Modulelementes aus Kunststoff aufgefangen, so daß ein Druck auf das obere Keramikteil, d.h. auf die Schleimhautmanschette, nicht mehr in der Weise ausgeübt werden kann, daß dieses zerbricht. Diese Ausgestaltung macht es möglich, das Implantat in seinen beiden keramischen Teilen durch sphärische Flächen aneinandergrenzen zu lassen, ohne daß die Keramikoberteile über Gebühr belastet werden und brechen können.

Weitere vorteilhafte Ausgestaltungen der Erfindung sind in den Unteransprüchen gekennzeichnet.

Ausführungsbeispiele der Erfindung werden nachstehend anhand der Zeichnungen erläutert. Es zeigt

Fig. 1 teils in Ansicht, teils in einem senkrechten Schnitt ein aus einem Primärzylinder und einem Sekundärzylinder mit einem als Schwingstab ausgebildeten Implantatpfosten bestehendes enossales Implantat,

Fig. 2 in einem senkrechten Schnitt den Primärzylinder nach Fig. 1,

Fig. 3 in einem senkrechten Schnitt den Sekundärzylinder nach Fig. 1,

Fig. 4 teils in Ansicht, teils in einem senkrechten Schnitt eine weitere Ausführungsform eines enossalen Implantates mit sphärischen Gleitflächen,

Fig. 5 in einem senkrechten Schnitt den Primärzylinder des Implantates nach Fig. 4,

Fig. 6 in einem senkrechten Schnitt den Sekundärzylinder des Implantates nach Fig. 4,

Fig. 7 teils in Ansicht, teils in einem senkrechten Schnitt eine weitere Ausführungsform eines enossalen Implantates mit einem Thermoverschluß,

Fig. 8 eine Detailvergrößerung des Übergangsbereiches Modulelement-Implantataufsatz-Sekundärzylinder bei der Ausführungsform gemäß Fig.7,

Fig. 9 teils in Ansicht, teils in einem senkrechten Schnitt eine weitere Ausführungsform eines aus einem Primärzylinder und einem Sekundärzylinder bestehenden enossalen Implantates mit einem in das Führungsrohr eingeklebten Implantatpfosten,

Fig. 10 in einem senkrechten Schnitt den Primärzylinder nach Fig. 9,

Fig. 11 in einem senkrechten Schnitt den Sekundärzylinder nach Fig.9,

Fig. 12 teils in Ansicht, teils in einem senkrechten Schnitt eine weitere Ausführungsform eines enossalen Implantates mit einem in das Führungsrohr eingeklebten Implantatpfosten und mit sphärischen Gleitflächen,

Fig. 13 in einem senkrechten Schnitt den Primärzylinder des Implantates nach Fig. 12,

Fig. 14 in einem senkrechten Schnitt den Sekundärzylinder des Implantates nach Fig. 12,

Fig. 15 teils in Ansicht, teils in einem senkrechten Schnitt eine weitere Ausführungsform eines enossalen Implantates mit eingeklebtem Implantatpfosten,

Fig. 16 eine Detailvergrößerung des Übergangsbereiches Modulelement-Implantataufsatz-Sekundärzylinder bei der Ausführungsform gemäß Fig. 15,

Fig. 17 teils in Ansicht, teils in einem senkrechten Schnitt eine weitere Ausführungsform eines enossalen Implantates mit einem in dem Primärzylinder mittels eines Bodenteils gehaltenen Implantatpfosten,

Fig. 18 teils in Ansicht, teils in einem senkrechten Schnitt ein enossales Implantat, bei dem der Implantatpfosten mittels eines lösbaren Klemmverschlusses in dem Primärzylinder gehalten ist,

Fig. 19 teils in Ansicht, teils in einem senkrechten Schnitt eine weitere Ausführungsform eines enossalen Implantates, bei dem der Implantatpfosten des Sekundärzylinders in dem Primärzylinder und der Montagekopf auf dem Implantatpfosten mittels lösbarer Klemmverschlüsse gehalten sind,

Fig. 20 teils in Ansicht, teils in einem senkrechten Schnitt den Sekundärzylinder des enossalen Implantates nach Fig. 19,

Fig. 21 teils in Ansicht, teils in einem senkrechten Schnitt den Primärzylinder des enossalen Implantates nach Fig. 19,

Fig. 22 in einem senkrechten Schnitt eine auf dem Implantatpfosten des Sekundärzylinders aufsetzbare Montagekappe,

Fig. 23 teils in Ansicht, teils in einem senkrechten Schnitt eine weitere Ausführungsform eines enossalen Implantates, bei dem der Implantatpfosten des Sekundärzylinders in dem Primärzylinder und der Montagekopf auf dem Implantatpfosten mittels unlösbarer Klemmverschlüsse gehalten sind,

Fig. 24 teils in Ansicht, teils in einem senkrechten Schnitt den Primärzylinder des enossalen Implantates nach Fig. 23,

Fig. 25 teils in Ansicht, teils in einem senkrechten Schnitt den Sekundärzylinder des enossalen Implantates nach Fig. 23,

Fig. 26 in einem senkrechten Schnitt die auf den Implantatpfosten des Sekundärzylinders aufsetzbare Montagekappe,

Fig. 27 teils in Ansicht, teils in einem senkrechten Schnitt ein enossales Implantat, bei dem der Implantatpfosten mittels eines unlösbaren Schnappverschlusses in dem Primärzylinder gehalten ist, und

Fig. 28 teils in Ansicht, teils in einem senkrechten Schnitt ein enossales Implantat mit einer Einrichtung zur Schaffung einer Spaltdichtigkeit im Bereich der sphärischen Flächen zwischen dem Primärzylinder und dem Implantataufsatz.

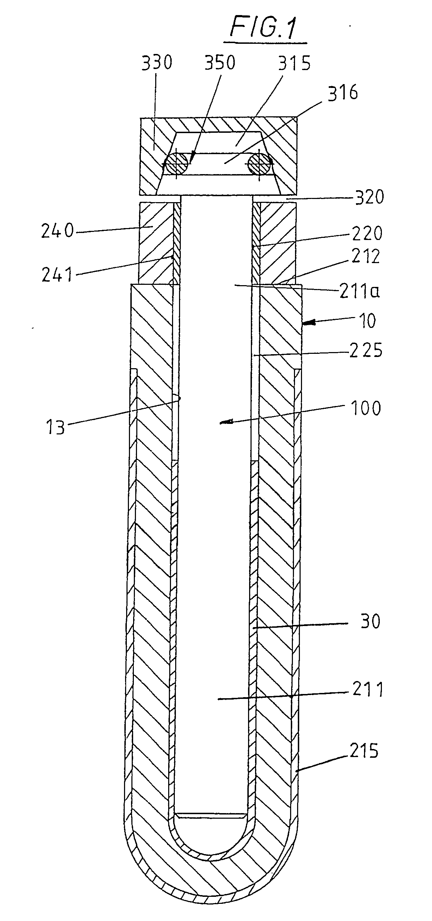

Das in Fig. 1 dargestellte enossale Implantat besteht aus einem Primärzylinder 10, dem sogenannten Aufnahmezylinder, und einem Sekundärzylinder 100, dem sogenannten Arbeitszylinder.

Der Primärzylinder 10 des enossalen Implantates besteht aus einem biegefesten Körper, insbesondere aus Aluminiumoxidkeramik und ist außenseitig mit einer Hydroxylapatitkeramik beschichtet, die mit 215 bezeichnet ist. Dieser Primärzylinder 10 ist der eigentliche Implantatkörper bzw. Werkstoffträger und weist eine mittige Längsbohrung 13 auf, die den Innenraum bildet (Fig.1).

Der Sekundärzylinder 100 wird nach der Implantation des Primärzylinders 10, d.h. etwa drei Monate nach der Implantation, in die Längsbohrung 13 des Primärzylinders 10 eingesetzt, wodurch die Verbindung zwischen dem Implantat und dem Zahnersatz hergestellt wird. In dem Innenraum bzw. in der Längsbohrung 13 des Primärzylinders 10 ist ein Führungsrohr 30 angeordnet, das sich in etwa über die gesamte Länge der Längsbohrung 13 erstreckt und u.a. das mühelose Einsetzen des Sekundärzylinders 100 in den Primärzylinder 10 ermöglicht.

Dieses Führungsrohr 30 gehört ebenfalls zum Kraftleitungssystem des enossalen Implantates, das, wie nachstehend näher beschrieben, aus Modulelementen aus verschiedenen Werkstoffen bestehen kann. Durch die elastische Verformung des aus hierzu geeigneten Werkstoffen bestehenden Führungsrohres 30 in dem Primärzylinder 10 kann ein zusätzlicher Kraftabbau erreicht werden, wobei die verbleibenden Kräfte in kristallografisch bestimmte Richtungen, z.B. in die unteren Bereiche des Primärzylinders 10, umgeleitet werden können. Bei der Verwendung und Herstellung des Führungsrohres 30 haben die sogenannten Einkristalle des verwendeten Werkstoffes, z.B. schwingungsfähiges Metall, als elastisch verformbare Umhüllung des im Primärzylinder 10 lagernden Sekundärzylinders 100 eine große technische Bedeutung. Vor allem ist es möglich, einkristalline Werkstoffe mit vorgegebenen Fehlstellen zu züchten und zu verwenden, wodurch die elastische Verformung des Führungsrohres 30 steuerbar ist, z.B. im Zusammenhang mit der Kraftübertragung von dem Sekundärzylinder 100 auf den Primärzylinder 10.

Der Sekundärzylinder 100 weist nach Fig.1 einen in die Bohrung 13 des Primärzylinders 10 einsetzbaren, mit 211 bezeichneten Implantatpfosten auf, so daß der Sekundärzylinder 100 auswechselbar ist. Der Durchmesser der zylindrischen Außenwand oder eines Teils der Außenwand des Implantatpfostens 211 des Sekundärzylinders 100 ist soviel größer als der Durchmesser der Längsbohrung 13 des Primärzylinders 10 bemessen, daß der Implantatpfosten 211 oder ein Teil des Implantatpfostens bei Körpertemperatur in der Längsbohrung 13 klemmend gehalten ist, jedoch bei einer Temperaturabsenkung aus der Längsbohrung des Primärzylinders lösbar bzw. herausziehbar ist. Eine solche Verbindung ist relativ einfach zu handhaben ; sie kann sich nicht lösen und ist praktisch spaltfrei, so daß sich keine Bakterienherde bilden und Entzündungen entstehen können.

Der Sekundärzylinder 100 selbst wird von dem Implantatpfosten 211 gebildet, dessen oberes freies Ende eine lösbare Verschlußvorrichtung für den Zahnersatz trägt. Des weiteren umfaßt der Sekundärzylinder 100 einen Implantataufsatz 240, der aus an sich bekannten Werkstoffen, wie z.B. Aluminiumoxidkeramik, besteht.

Der Sekundärzylinder 100 umfaßt als Schwingstab den Implantatpfosten 211, auf dem ein oberes rohrförmiges Modulelement 220 angeordnet ist, das aus einem stark elastischen Werkstoff, der auch metallischer Art sein kann, jedoch auch aus Kunststoff oder einem anderen geeigneten Werkstoff bestehen kann. Dieses Modulelement 220 ist im oberen Bereich des Implantatpfostens 211 des Sekundärzylinders 100 angeordnet und vermittels einer Klebeverbindung auf diesem gehalten.

Im Innenraum der Längsbohrung 13 des Primärzylinders 10, und zwar in einem Abstand von der Öffnung 211a der Längsbohrung 13 ist das Führungsrohr 30 angeordnet. Der Abschnitt zwischen dem Modulelement 220 und dem Führungsrohr 30 ist modulementfrei und bildet bei in den Primärzylinder 10 eingesetztem Sekundärzylinder 100 einen Luftspalt, der bei 225 angedeutet ist (Fig.1).

Das Führungsrohr 30, welches sich über einen größeren Bereich der Länge des Primärzylinders 10 erstreckt, ist in dem Primärzylinder 10 mittels einer Klebeverbindung fest angeordnet.

Der Sekundärzylinder 100 besteht aus dem in die Längsbohrung 13 des Primärzylinders 10 einsetzbaren Implantatpfosten 211 aus geeigneten Werkstoffen und einer oberen kopfartigen Erweiterung 315, dem Schwingkopf, der den Montagekopf bildet und eine Montagekappe 330 aufnimmt, die auf dem Schwingkopf 315 lösbar gehalten ist.

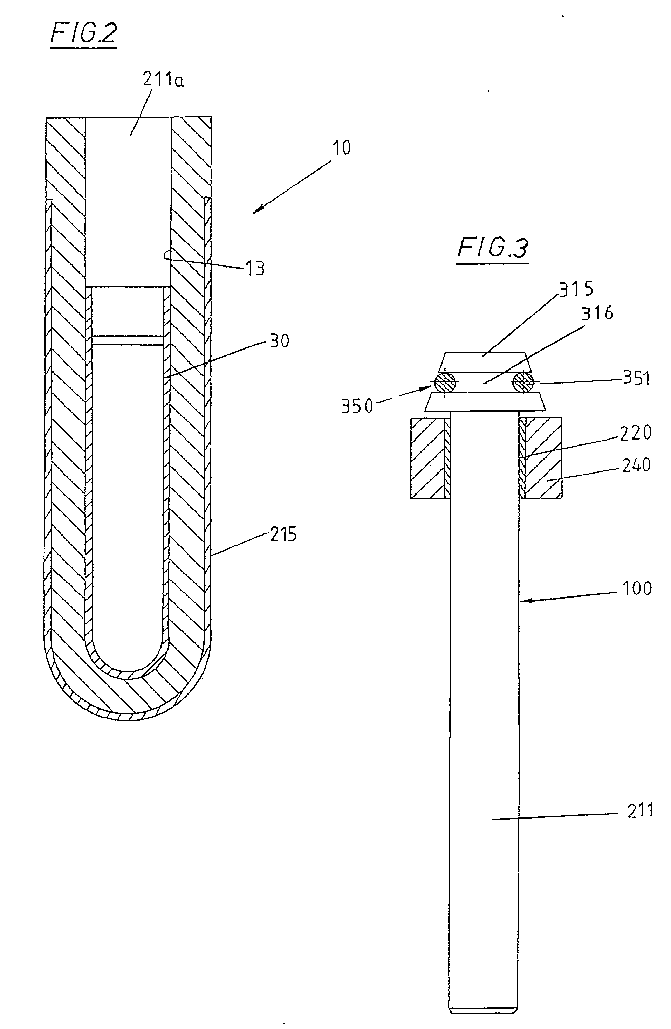

Das Modulelement 220 erstreckt sich nur im Bereich des Implantataufsatzes 240. Auf das Modulelement 220 ist dieser Implantataufsatz 240 aufgesetzt, der mit einer dem Außendurchmesser des rohrförmigen Modulelementes 220 entsprechenden, mittigen Durchbohrung 241 versehen ist. Bei der in Fig. 1 und 3 gezeigten Ausführungsform weist der Implantataufsatz 240 eine zylindrische Form und einen Außendurchmesser auf, der in etwa dem Außendurchmesser des Primärzylinders 10 entspricht; jedoch kann der Außendurchmesser des Implantataufsatzes 240 auch gegenüber dem Außendurchmesser des Primärzylinders 10 einen geringeren Durchmesser aufweisen, so daß dann der Außendurchmesser des Implantataufsatzes 240 mit dem Außendurchmesser der Montagekappe 330 übereinstimmt. Diese Montagekappe 330 besteht aus metallischen Werkstoffen, insbesondere edelmetallischen Werkstoffen.

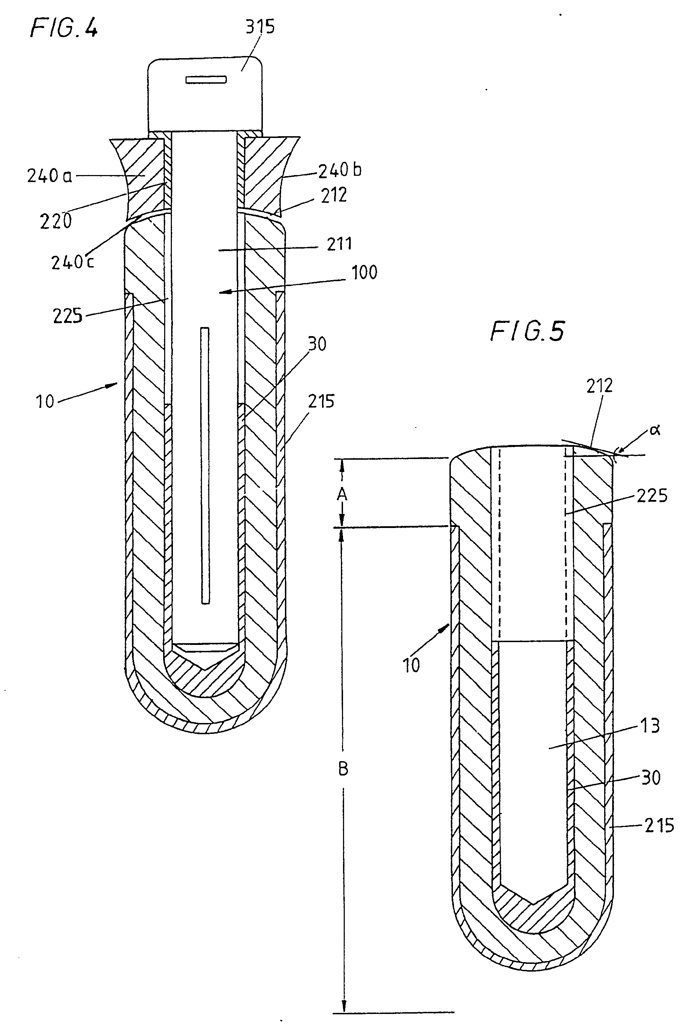

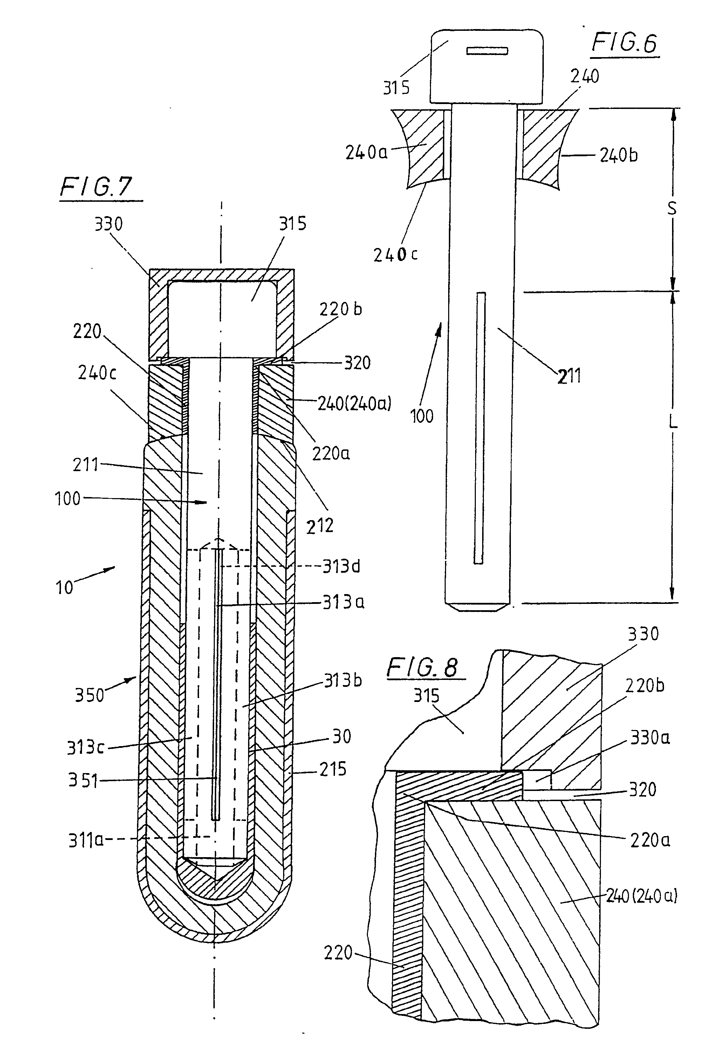

Während bei dem in Fig. 1 und 2 gezeigten Ausführungsbeispiel des Implantats die Gleitfläche 212 im oberen Bereich des Primärzylinders 10 senkrecht zur Längsachse des Primärzylinders 10 und somit waagerecht verlaufend ist, ist die Gleitfläche 212 des Primärzylinders 10 für den Implantataufsatz 240a nach der Ausführungsform gemäß Fig.4 und 5 als Kalotte mit einem Winkel $\alpha$ von insbesondere 9,27° ausgebildet. Bei dieser Ausführungsform ist der obere, mit 240a bezeichnete Implantataufsatz (Fig.6) als etwa zylindrischer Formkörper ausgebildet, dessen Außenwandfläche 240b kreisbogenförmig eingezogen ist und dessen unterer Außendurchmesser etwa dem Außendurchmesser des Primärzylinders 10 entspricht, während der obere Außendurchmesser des Implantataufsatzes 240a gegenüber dem unteren Außendurchmesser größer bemessen ist. Des weiteren ist die untere Auflagefläche 240c des Implantataufsatzes 240a der Gleitfläche 212 des Primärzylinders 10 anpassend ausgebildet, so daß ein einwandfreies Gleiten des Implantataufsatzes 240a auf dem Primärzylinder 10 gewährleistet ist. Damit ist der Implantataufsatz 240a auf der Oberfläche des Primärzylinders 10 gleitend gehalten.

Der Implantatpfosten 211 des Sekundärzylinders 100 ist in dem Führungsrohr 30 verankert. Der Außendurchmesser des Implantatpfostens 211 ist kleiner als der Innendurchmesser der Längsbohrung 13 des Primärzylinders 10, so daß der Implantatpfosten 211 unter festem Sitz in der Längsbohrung 13 des Primärzylinders 10 gehalten ist.

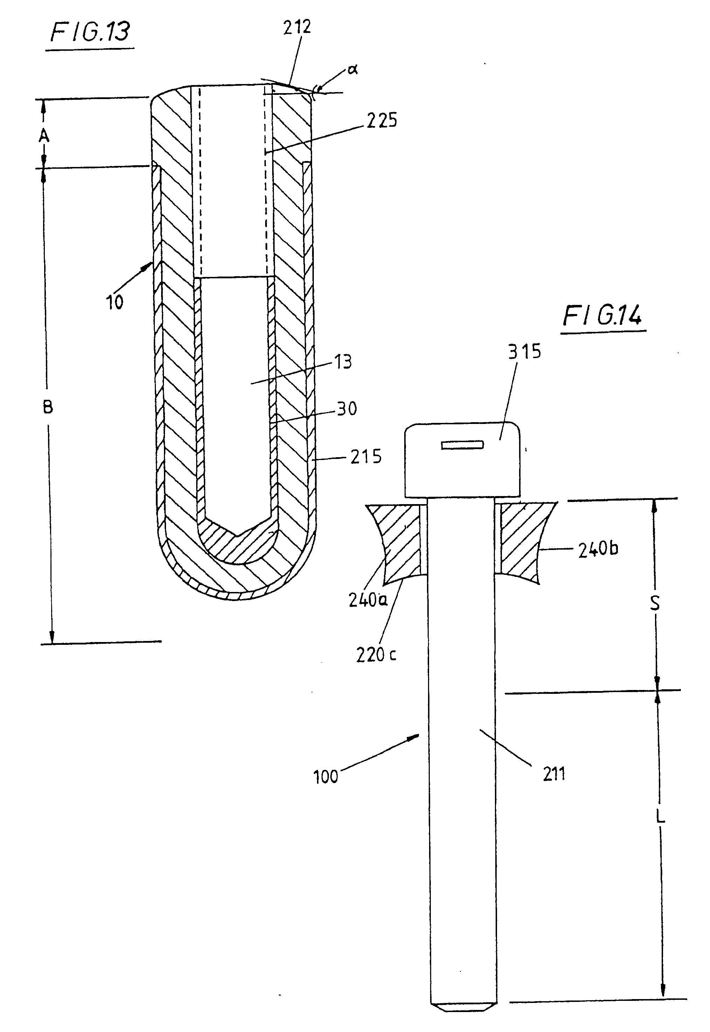

Des weiteren ist der Implantatpfosten 211 des Sekundärzylinders 100 mittels eines Thermoverschlusses in dem Primärzylinder 10 gehalten. Dieser Thermoverschluß besteht aus einem Draht aus Bimetall, z.B. Memorymetall, der in einer Ringnut gehalten ist, die in dem Implantatpfosten 211 ausgebildet ist. In gleicher Weise, wie der Implantatpfosten 211 mittels Thermoverschlusses in dem Primärzylinder 10 gehalten ist, ist auch die Montagekappe 330 auf dem Montagekopf 315 gehalten. Hier ist der Thermoverschluß mit 350 bezeichnet und besteht ebenfalls aus einem Draht 351 aus Bimetall, z.B. Memorymetall, wobei dieser Draht 351 in einer Ringnut 316 in dem Montagekopf 315 gehalten ist. Der Montagekopf 315 ist konisch sich nach oben verjüngend ausgebildet (Fig.3). Die Außenbeschichtung 215 an dem Primärzylinder 10 besteht insbesondere aus einer Hydroxylapatitkeramik. Wie Fig. 5 zeigt, erstreckt sich die Außenbeschichtung 215 nur über einen Teilbereich des Primärzylinders 10, und zwar über den größeren Bereich des Primärzylinders. Der obere Bereich des Primärzylinders ist außenbeschichtungsfrei. Dieser außenbeschichtungsfreie Abschnitt stellt die Kontaktzone A dar, die der Kortikalis entspricht, während die der Spongiosa entsprechende Kontaktzone B diese Außenbeschichtung 215 trägt (Fig. 13).

Zum Abbau des Drehmomentes bringen dynamisch an der Montagekappe 330 und damit auf den Montagekopf 315 des Implantatpfostens 211 des Sekundärzylinders 100 übertragene Horizontalkräfte den Implantatpfosten 211 zum Schwingen. Es entsteht ein Drehmoment. Dieses Drehmoment wird über den Implantatpfosten abgebaut, und zwar wie folgt:
Der Implantataufsatz 240 bzw. 240a gleitet auf der Oberfläche 212 des Primärzylinders 10. Ein Teil des Drehmomentes wird durch die hier entstehende Gleitreibung verbraucht. Bei dem Implantat nach Fig 4 ist diese Gleitfläche 212 als Kalotte ausgebildet, und zwar vorteilhafterweise mit einem Winkel $\alpha$ von 9,27°. Da der Implantataufsatz 240, 240a auf dem Primärzylinder 10 in diesem Bereich 240c gleitet, können keine Verkantungen und keine Ausbrüche auftreten, wie dies bei exakt planen Flächen und den hohen Flächenpressungen erfolgen kann, wenn diese vorgesehen sind. Die Flächen gleiten dabei nicht trocken, sondern feucht aufeinander. Diese Feuchtigkeit gewährleistet der verwendete Werkstoff Aluminiumoxidkeramik. Dieser hat die Eigenschaft, an seiner Oberfläche freie Sauerstoffionen zur Verfügung zu stellen, die sich mit Substanzen aus dem umliegenden Milieu automatisch zu einem freien Schmierfilm verbinden. Das Material selbst sorgt so für eine Selbstschmierung auf der Gleitfläche. Diese Eigenschaft der Aluminiumoxidkeramik führt zu einem hohen Selbstschmiereffekt.

Das obere Modulelement 220 ist aus einem stark elastischen Material hergestellt und wirkt wie ein Schwingungsdämpfer. Durch diese Dämpfung wird ein weiterer Teil des Drehmomentes in Wärme abgebaut. Unter dem oberen Modulelement 220 befindet sich der Luftspalt 225, so daß in seinem Schwingbereich der Implantatpfosten 211 des in den Primärzylinder eingesetzten Sekundärzylinders 100 frei schwingen kann. Die dabei auftretenden Schwingungen werden in Wärme abgebaut.

Bei der in Fig. 4 und 6 gezeigten Ausführungsform weist der Implantataufsatz 240a eine andere Form gegenüber dem Implantataufsatz 240 auf. Der Implantataufsatz 240a ist als etwa zylindrischer Formkörper ausgebildet, dessen Außenwandfläche 240b kreisbogenförmig eingezogen ist und dessen unterer Außendurchmesser etwa dem Außendurchmesser des Primärzylinders 10 entspricht, während der obere Außendurchmesser einen gleichen oder größeren Außendurchmesser als der Primärzylinder 10 aufweist.

Unterhalb des Luftspaltes 225 befindet sich das Führungsrohr 30 in dem Innenraum des Primärzylinders 10. Dieses Führungsrohr 30 ist in den Primärzylinder eingeklebt. Der Implantatpfosten des Sekundärzylinders 100 ist paßgenau in dem Führungsrohr 30 verankert. Im oberen Bereich des Führungsrohres 30 befindet sich auf dem Implantatpfosten 211 der Thermoverschluß 250.

Durch die Verankerung mittels des Thermoverschlusses 250 und durch die Einpassung in das Führungsrohr 30 werden nicht auf vorgeschriebene Weise abgebaute Drehmomente im gesamten Lagerbereich des Implantatpfostens 211 über das Führungsrohr 30 auf den Primärzylinder 10 übertragen, und zwar nicht punktuell im Bereich des Thermoverschlusses 250, sondern verteilt über den Gesamtbereich des Führungsrohres 30. Die verbleibenden, auf diese Weise verteilt an den Knochen abgegebenen Drehmomente sind dabei so geringfügig, daß sie den über die Hydroxylapatitbeschichtung 215 des Außenzylinders des Primärzylinders 10 hergestellten festen Verbund des Primärzylinders 10 mit dem Knochen nicht mehr beeinträchtigen. Diese "energie -abbauende" und "lagerschonende" Mechanik macht es daher erst möglich, Primärzylinder aus Aluminiumoxidkeramik von einer Wandstärke von nur 0,9 mm einzusetzen und damit erstmalig ein aus körperfreundlicher Aluminiumoxidkeramik hergestelltes Spätimplantat von nur 4 mm Durchmesser herzustellen, weil eben durch die drehmomentabbauende Mechanik der Primärzylinder 10 kaum noch mechanisch beansprucht wird. Wesentlich trägt hierzu bei die Ausgestaltung des Modulelementes 220 aus einem stark elastischen Werkstoff im Bereich des Implantataufsatzes 240, der sich an das Modulelement 220 anschließende Luftspalt 225 und das den Implantatpfosten 211 umgebende Führungsrohr 30 aus einem gegenüber dem Modulelement 220 eine geringere Elastizität aufweisenden Werkstoff.

Zwischen dem Schwingkopf 315 des Implantatpfostens 211 des Sekundärzylinders 100 – Unterseite des Konus – und dem Implantataufsatz 240 befindet sich eine Luftschicht 320 (Fig.1). Der Implantatpfosten 211 kann somit infolge horizontal einwirkender Kräfte schwingen. Er wird gedämpft durch das obere Modulelement 220, das bei eingesetztem Sekundärzylinder 100 sich mit einem Abschnitt in den Innenraum der Längsbohrung 13 des Primärzylinders 10 erstreckt. Aufgrund dieser Ausgestaltung gibt das Modulelement 220 gleichzeitig die Führung in den Primärzylinder 10 ab. Der Implantataufsatz 240 gleitet auf der Gleitfläche, die von der Unterseite des Implantataufsatzes 240 und der Oberseite des Primärzylinders 10 gebildet wird. Beide Flächen sind plan geschliffen und hochglanzpoliert. Bei horizontal ansetzenden Kaukräften auf den Kopf 315 des Implantatpfostens 211 bzw. die darüber liegende Montagekappe 330 bewegt sich mithin der Sekundärzylinder 100 auf dem Primärzylinder 10. Die horizontale Verschiebung ist dabei auf 150 μ ausgelegt, was der Beweglichkeit des natürlichen Parodontiums entspricht.

Die Mechanik, die die Imitation des natürlichen Parodontiums hervorruft, ist absolut wartungsfrei.

Wesentlich ist ferner, daß das Implantat aufgrund seiner konstruktiven Ausgestaltung eine hohe Spaltdichtigkeit gewährleistet. Das Implantat selbst besteht aus nur zwei Teilen, nämlich dem Primärzylinder 10 und dem Sekundärzylinder 100. Der dabei verwendete Thermoverschluß 250 besteht aus dem Draht 251 aus Memorymetall, der in der Ringnut 312 verläuft, die in den Implantatpfosten 211 eingelassen ist. Durch Ausdehnung bei Körpertemperatur verankert der Thermoverschluß 250 den Implantatpfosten 211 und damit den Sekundärzylinder 100 in dem Primärzylinder 10. Das Führungsrohr 30 ist für das Einrasten des Thermoverschlusses mit einer Nut versehen. Diese Nut bildet in ihrem oberen Teil eine schiefe Ebene und in ihrem unteren Teil eine Rundung, so daß bei Ausdehnung des umlaufenden Drahtes 251 aus Memorymetall der Sekundärzylinder 100 in den Primärzylinder 10 hineingezogen wird. Es entsteht dadurch im Bereich der Gleitflächen zwischen dem Implantataufsatz 240 und dem Primärzylinder 10 eine Spaltdichte, die sowohl bakterien- als auch feuchtigkeitsdicht ist.

Das Führungsrohr 30 ist unter definiertem Druck in dem Primärzylinder 10 verklebt, in dem der Luftspalt 320 zwischen dem Schwingkopf 315 des Implantatpfostens 211 und der Montagekappe 330 und dem Implantataufsatz 240 durch eine vorgesehene unelastische Scheibe überbrückt wird und das Führungsrohr 30 so plaziert ist, daß der Thermoverschluß 250 maximal die definierte Zugkraft entwickelt. Der Zahnarzt braucht dann den Sekundärzylinder 100 nur noch unter gleich definiertem Druck einzubringen, um sicherzustellen, daß der Thermoverschluß an vorgesehener Stelle in die Nut einrastet und damit der vorgegebene Zug und die Spaltsicherheit sichergestellt ist. Die Einbringung selbst erfolgt durch Biß des Patienten auf die Montagekappe 330 unter Verwendung von Druckmeßstreifen, die an eine Digital-Druckablese-Vorrichtung angeschlossen sein können. Die voranstehend erwähnte Spaltdichtigkeit wird insbesondere durch die Verwendung der beiden Thermoverschlüsse 250, 350 entsprechend der in Fig. 1 gezeigten Ausführungsform erreicht.

Eine weitere Ausführungsform des Implantates ist in den Fig. 7 und 8 dargestellt, dessen Grundaufbau der Ausführungsform gemäß Fig. 4 entspricht. Das Modulelement 220 ist hierbei in seinem oberen Endbereich 220a mit einem auskragenden, scheiben- oder ringförmigen Abschnitt 220b versehen, der den Implantataufsatz 240, 240a teilweise übergreift, wobei der Abschnitt 220b sich radial über den Außendurchmesser des Schwingkopfes 315 hinaus bis in den Bereich einer Eingriffsnut 330a in der Montagekappe 330 erstreckt und gleichzeitig die Ausbildung eines Luftspaltes 320 sicherstellt. Auf diese Weise wird es möglich, das Modulelement 220 sowohl sicher mit dem Implantataufsatz 240, 240a als auch mit dem Schwingkopf 315 zu verkleben und damit sicher zu verbinden und gleichzeitig eine Ausbildung des Luftspaltes 320 sicherzustellen (Fig. 8).

Nach Fig. 7 ist eine von den anderen Ausführungsformen eines Thermoverschlusses des Implantates abweichende Ausführungsform eines Thermoverschlusses vorgesehen. Hierzu ist der Implantatpfosten 211 mit zur Längsachse des Implantatpfostens parallelen, in den Innenraum einer Längsdurchbohrung 311a des Implantatpfostens 211 hineinreichenden Längsdurchbrechungen 313a, 313b, 313c, 313d versehen. Dabei werden die Drähte aus einem Bimetall, z.B. einem Memorymetall, durch die Längsdurchbohrung 311a in den Innenraum des Implantatpfostens 211 derart eingeführt, daß sie durch die Längsdurchbrechungen 313a, 313b, 313c, 313d nach außen hindurchreichen und bei in den Primärzylinder 10 eingesetztem Sekundärzylinder 100 mit dem Führungsrohr 30 des Primärzylinders 10 in Kontakt stehen.

Auf diese Weise ist ebenfalls sichergestellt, daß der Implantatpfosten 211 im Lagerbereich sicher in dem Führungsrohr 30 gehalten ist, so daß hier eine großflächige Energieübertragung der nicht schon bereits im Schwingbereich in Wärme verwandelten Energie auf den Primärzylinder 10 ermöglicht wird.

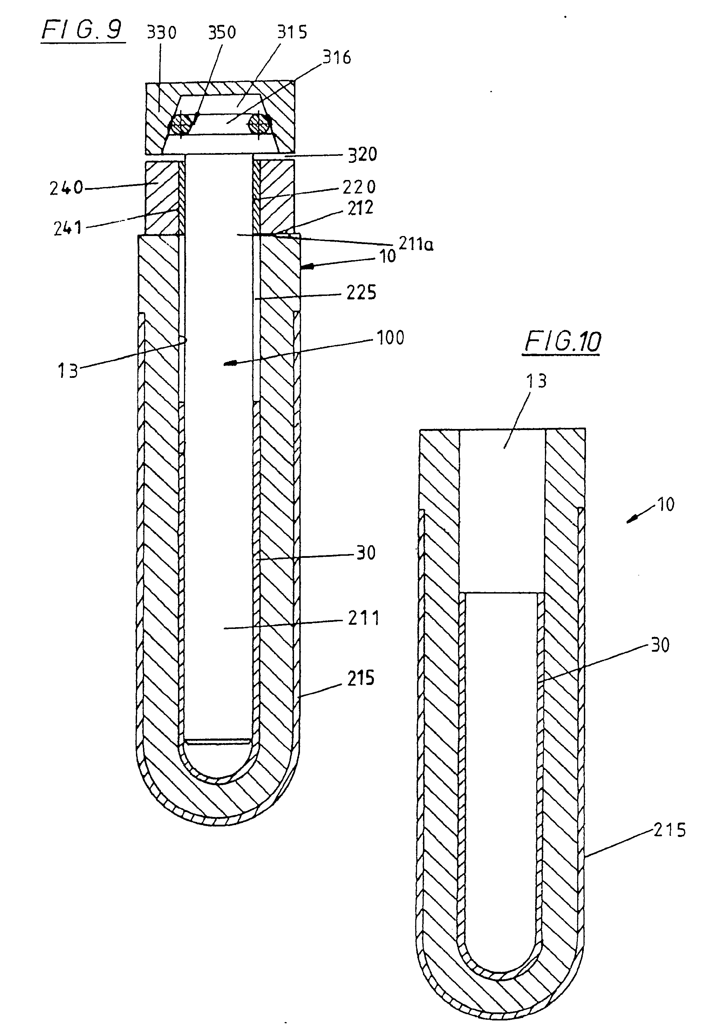

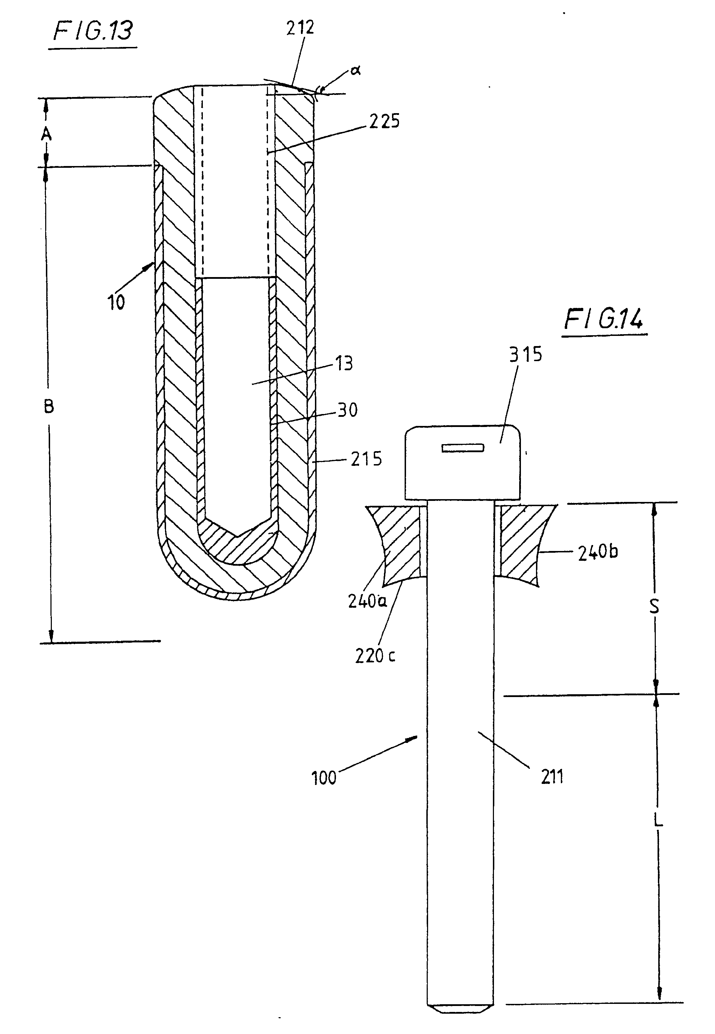

Das in den Fig. 9 bis 16 dargestellte enossale Implantat besteht ebenfalls aus dem Primärzylinder 10 und dem Sekundärzylinder 100 und ist entsprechend dem Implantat nach den Fig. 1 bis 8 ausgebildet, jedoch weist das Implantat dieser weiteren Ausführungsform in dem Implantatpfosten 211 keinen Thermoverschluß 250 auf, wobei für gleiche Teile

gleiche Bezugzeichen in den Fig. 9 bis 16 verwendet wurden. Doch wird der Sekundärzylinder 100 vor der Implantation des gesamten Implantats in den Primärzylinder 10 eingesetzt.

Des weiteren ist der Implantatpfosten 211 des Sekundärzylinders 100 mittels einer Klebeverbindung in dem Primärzylinder 10 gehalten. Dabei ist der Implantatpfosten 211 mit seinem bodenseitigen Ende mittels einer Klebeverbindung an dem Führungsrohr 30 befestigt. Es besteht jedoch auch die Möglichkeit, den Primärzylinder 10 unter Weglassung des Führungsrohres 30 auszubilden, so daß dann der Implantatpfosten 211 mit seinem bodenseitigen Ende direkt an der Wandfläche der Längsbohrung 13 des Primärzylinders 10 mittels der Klebeverbindung gehalten ist.

Es besteht darüber hinaus auch die Möglichkeit, den Implantatpfosten 211 des Sekundärzylinders 100 etwa in der Mitte des Primärzylinders 10 oder des Führungsrohres 30 anzuordnen und mit dem Führungsrohr 30 oder der Wandfläche der Längsbohrung 13 des Primärzylinders 10 mittels der Klebeverbindung zu befestigen, wobei jedoch der Verbindungsbereich sich nur über einen kurzen Abschnitt zu erstrecken braucht.

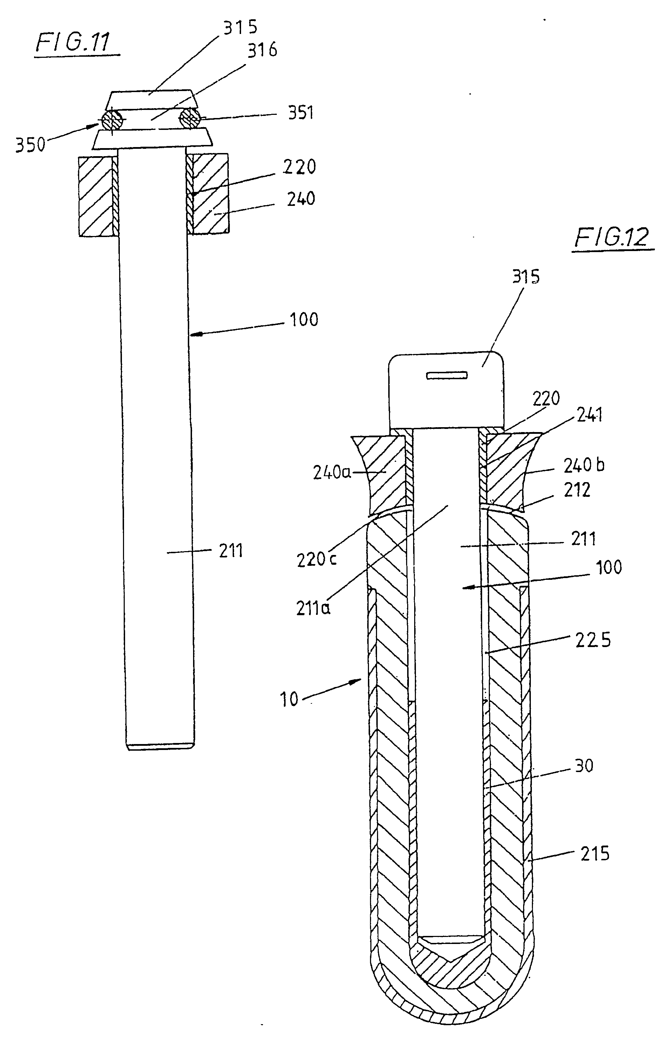

Die Montagekappe 330 kann auf dem Schwingkopf 315 mittels eines Thermoverschlusses befestigt sein, der in Fig. 9 mit 350 bezeichnet ist. Dieser Thermoverschluß 350 besteht aus einem Draht 351 aus Bimetall, wie z.B. Memorymetall, der in einer Ringnut 316 in dem Montagekopf 315 gehalten ist. Der Montagekopf 315 ist konisch sich nach oben verjüngend ausgebildet (Fig. 11).

Unterhalb des Luftspaltes 225 befindet sich das Führungsrohr 30 in dem Innenraum des Primärzylinders 10. Dieses Führungsrohr 30 ist in den Primärzylinder 10 eingeklebt. Der Implantatpfosten 211 des Sekundärzylinders 100 ist paßgenau in dem Führungsrohr 30 verankert und ebenfalls in diesem mittels einer Klebeverbindung gehalten, wobei sich die Klebeverbindung auch über den ganzen Bereich des Führungsrohres 30 erstrecken kann.

Durch die Verankerung des Implantatpfostens 211 mittels der Klebeverbindung und durch die Einpassung in das Führungsrohr 30 werden auch bei dieser Ausführungsform nicht auf vorgeschriebene Weise abgebaute Drehmomente im gesamten Lagerbereich des Implantatpfostens 211 über das Führungsrohr 30 auf den Primärzylinder 10 übertragen, und zwar nicht punktuell im Bereich der Klebeverbindung, sondern verteilt über den Gesamtbereich des Führungsrohres 30. Die verbleibenden, auf diese Weise verteilt an den Knochen abgegebenen Drehmomente sind auch hier so geringfügig, daß sie den über die Hydroxylapatitbeschichtung 215 des Außenzylinders des Primärzylinders 10 hergestellten festen Verbund des Primärzylinders mit dem Knochen nicht mehr beeinträchtigen. Das Führungsrohr 30 ist dabei unter definiertem Druck mit dem Primärzylinder 10 in diesem verklebt, indem der Luftspalt 320 zwischen dem Schwingkopf 315 des Implantatpfostens 211 und der Montagekappe 330 und dem Implantataufsatz 240 durch eine vorgesehene unelastische Scheibe überbrückt wird.

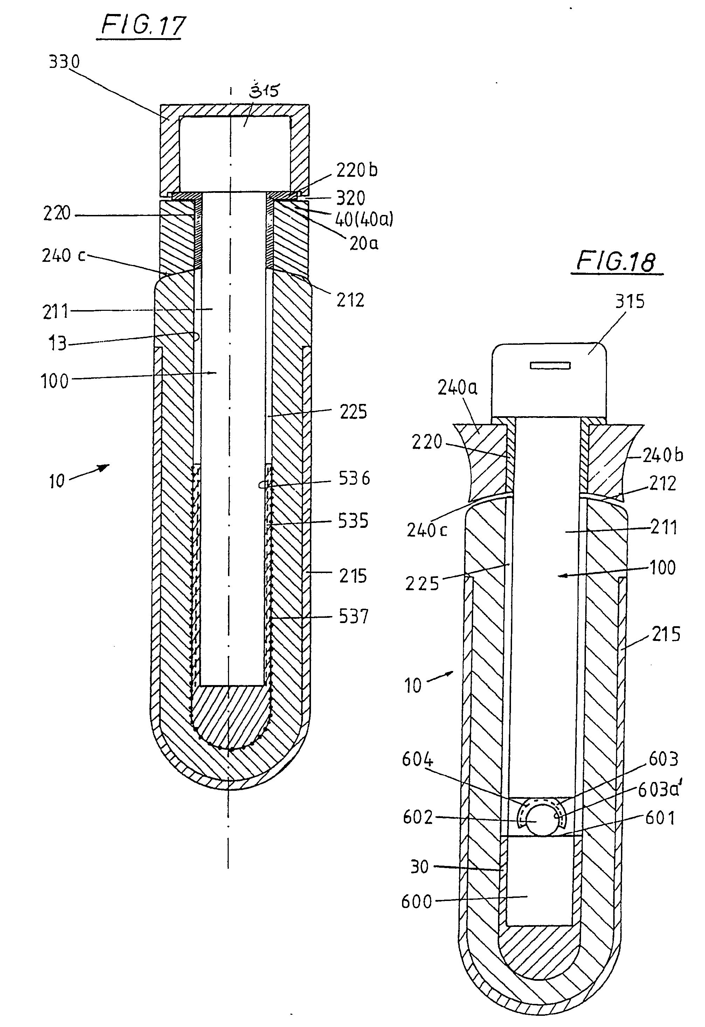

Fig. 17 zeigt ein Implantat, bestehend aus dem Primärzylinder 10 und dem Sekundärzylinder 100, dessen Implantatpfosten 211 in dem Innenraum des Primärzylinders mittels eines Schraubgewindes 536 in einem Formkörper 535 befestigt ist, der mittels einer bei 537 angedeuteten Klebeverbindung in dem Innenraum des Primärzylinders befestigt ist. Bei dieser Ausführungsform ist kein Führungsrohr vorgesehen, sondern der Formkörper 535 weist eine Ausgestaltung auf, aufgrund der der sonst von dem Führungsrohr eingenommene Hohlraum ausgefüllt wird, und zwar einschließlich des zwischen dem bodenseitigen Ende des Implantatpfostens 211 und der inneren Bodenfläche des Primärzylinders 10 ausgebildeten Hohlraums. Der Formkörper 535 weist eine obenseitige Bohrung auf, die mit einem Innengewinde versehen ist, in das das Außengewinde am Ende des Implantatpfostens 211 eingreift, wodurch dann die Schraubverbindung 536 nach dem Einsetzen des Implantatpfostens 211 in den Formkörper 535 hergestellt wird. Ansonsten ist das Implantat entsprechend der Ausführungsform gemäß Fig. 15 ausgebildet und mit dem Implantataufsatz 240, 240a und dem Schwingkopf 315 versehen, der von der Montagekappe 330 umgriffen wird. Der obere Bereich des Implantatpfostens 211 stützt sich an der Innenwandfläche des Modulelementes 220 ab.

Bei den vorangehend beschriebenen Beispielen bestehen der Primärzylinder 10 und der Sekundärzylinder 100 aus keramischen Werkstoffen. Der Primärzylinder 10 ist durch die Kortikalis in die Spongiosa eingebracht, wobei die Primärstabilität durch Formschlüssigkeit zwischen dem Implantat und dem Implantatbett einerseits und durch die Kraftschlüssigkeit der nachgewiesenen Anlagerung von Knochengewebe an die Aluminiumoxidkeramik-Oberfläche des Primärzylinders andererseits erreicht wird.

Anstelle von Thermoverschlüssen oder anderen Befestigungssystemen für den Implantatpfosten 211 in dem Primärzylinder 10 sind auch lösbare oder unlösbare Klemmverschlüsse einsetzbar, wie diese in Fig. 18 bis 27 dargestellt sind.

Bei der in Fig. 18 gezeigten Ausführungsform ist der Implantatpfosten 211 des Sekundärzylinders 100 mittels eines lösbaren Klemmverschlusses in dem Primärzylinder 10 gehalten. Hierzu weist das im bodenseitigen Bereich mittels einer Klebeverbindung in dem Primärzylinder 10 gehaltene Führungsrohr 30 eine kurze Länge auf, in dessen Innenraum ein als Vollzylinder ausgebildeter Formkörper 600 aus einem metallischen Werkstoff oder einem anderen geeigneten Werkstoff angeordnet ist, der mittels einer Klebeverbindung mit dem Führungsrohr 30 fest verbunden ist. Dieser Formkörper 600 weist vorteilhafterweise eine der Länge des Führungsrohres 30 entsprechende Länge auf.

Der Formkörper 600 ist auf seiner dem Moduleelement 220 zugekehrten Seite 601 mit einer kugelförmigen oder eine andere geeignete geometrische Form aufweisenden Halterung 602 versehen. Diese Halterung 602 wird von einem kapselförmigen Element 603 umgriffen, das am bodenseitigen Ende des Implantatpfostens 211 an diesem angeformt ist. Der Formkörper 600 mit seiner Halterung 602 ist einstückig ausgebildet. In gleicher Weise ist auch das Element 603 an dem bodenseitigen Ende des Implantatpfostens 211 ausgebildet und mit diesem fest verbunden.

Dieses kapselförmige Element 603 an dem Implantatpfosten 211 trägt an seiner der Halterung 602 zugekehrten Innenwandfläche 603a einen Bimetall-Streifen 604, der z.B. aus Memorymetall besteht. Dieser Bimetall-Streifen 604 kann in das Material des kapselförmigen Elementes 603 eingearbeitet sein ; es besteht jedoch auch die Möglichkeit, den Bimetall-Streifen 604 in Form einer Innenbeschichtung oder als Einlage an der Innenwandfläche des kapselförmigen Elementes 603 anzuordnen. Die gesamte Halterung 602 kann ebenfalls aus Memorymetall bestehen.

Das kapselförmige Element 603 wird im unterkühlten Zustand des Bimetall-Streifens bzw. des verwendeten Memorymetalls auf die kugelförmige Halterung 602 aufgesetzt, so daß bei einem Erwärmen bzw. Erhitzen des Bimetall-Streifens, d.h. des kapselförmigen Elementes 603, dieses Element die kugelförmige Halterung 602 umgreift, so daß der in den Primärzylinder 10 eingesetzte Implantatpfosten 211 des Sekundärzylinders 100 fest an dem Formkörper 600 gehalten wird, jedoch die Möglichkeit besteht, aufgrund dieser konstruktiven Ausgestaltung der Klemmverbindung den Sekundärzylinder 100 aus dem Primärzylinder 10 herauszuziehen. Die Halterung 602 kann auch aus einem federnd-elastischen Werkstoff bestehen.

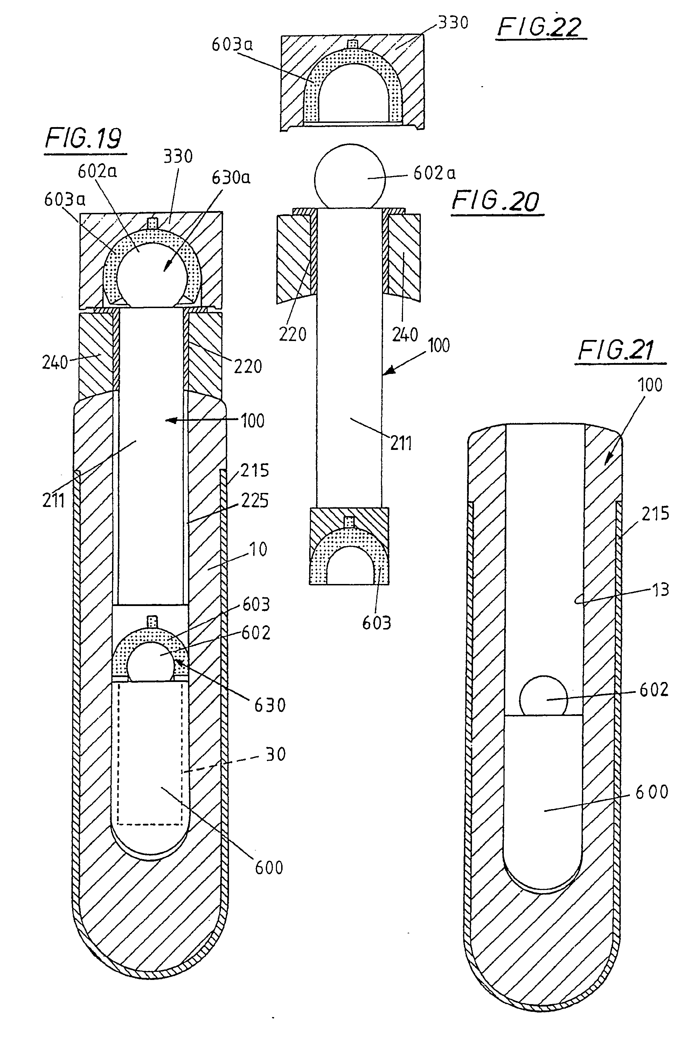

Nach Fig. 19 bis 22 ist sowohl der Implantatpfosten 211 des Sekundärzylinders 100 in den Primärzylinder 10 als auch der Montagekopf 330 auf dem Implantatpfosten 211 mittels eines Klemmverschlusses aus einem Memorymetall gehalten. Diese beiden Klemmverschlüsse sind in Fig. 19 bei 630, 630a angedeutet. Bei dieser Ausführungsform des enossalen Implantates ist kein Führungsrohr 30 in der Längsbohrung des Primärzylinders 10 vorgesehen. Im bodenseitigen Bereich des Primärzylinders 10 ist in dessen Längsbohrung der Formkörper 600 angeordnet, der mit der Innenwandfläche des Primärzylinders mittels einer Klebeverbindung verbunden ist. Dieser Formkörper 600 trägt in seinem oberen Bereich die kugelförmige Halterung 602, die von dem aus Memorymetall bestehenden, kapselförmigen Element 603 umgriffen wird, das am unteren Ende des Implantatpfostens 211 an diesem befestigt ist. Entsprechend dem Klemmverschluß 630 ist auch der Klemmverschluß 630a ausgebildet, mit dem der Montagekopf 330 auf dem Implantatpfosten 211 gehalten ist (Fig. 19, 20 und 22). Dieser Klemmverschluß 630 wird gebildet von der kugelförmigen Halterung 602a, die am oberen Ende des Implantatpfostens 211 an diesem ausgebildet ist. Das Gegenelement, das kapselförmige Element 603a aus Memorymetall, ist in dem Montagekopf 330 selbst ausgebildet. Fig. 22 zeigt das kapselförmige Element 603a vor der Verformung der Eingriffstellung.

Nach einer weiteren Ausführungsform kann anstelle des Bimetall-Streifens 604 das kapselförmige Element 603 an seiner Innenwandfläche mit einer Beschichtung, Einlage od. dgl. aus einem dauerelastischen Kunststoff, wie z.B. einem Polyurethan-Elastomer oder einem anderen geeigneten elastischen Werkstoff, versehen sein. Durch die Verwendung eines Bimetall-Streifens wird erreicht, daß, wenn sich dieser ausdehnt, dann der Sekundärzylinder 100 gegen den Primärzylinder 10 gezogen wird, so daß über diesen Weg die Reibung der sphärischen Gleitflächen des Sekundärzylinders 100 und des Implantataufsatzes 240 dosierbar ist. Im übrigen ist der Aufbau des Implantates gem. Fig.18 entsprechend dem des in Fig. 4 dargestellten Implantates. Das Führungsrohr 30 braucht nicht die in Fig.18 gezeigte kurze Länge aufweisen ; es besteht auch die Möglichkeit, ein Führungsrohr 30 mit der in Fig. 4 gezeigten Länge zu verwenden, wobei dann der Formkörper 600 in dem bodenseitigen Bereich des Führungsrohres 30 in diesem mittels einer Klebeverbindung befestigt ist, der gegenüber dem Formkörper 600 dann auch auch eine kürzere Länge aufweisen kann.

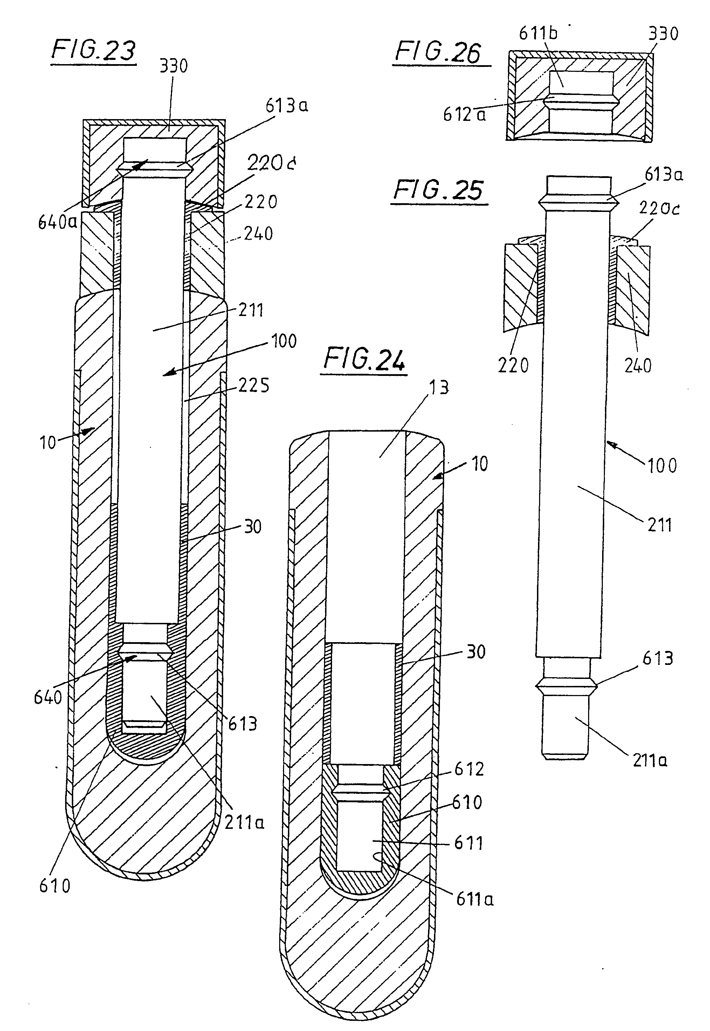

Bei dem in den Fig. 23 bis 26 gezeigten Ausführungsbeispiel ist der Implantatpfosten 211 mittels eines unlösbaren Klemmverschlusses 640 in dem Primärzylinder 10 gehalten. Hierzu ist das im unteren Bereich des mittels einer Klebeverbindung in dem Primärzylinder 10 befestigte Führungsrohr 30 in seinem bodenseitigen Bereich als Vollzylinder ausgebildet. Der so geschaffene Formkörper ist mit 610 bezeichnet und besteht aus einem metallischen Werkstoff oder einem anderen geeigneten Werkstoff. Dieser Formkörper 610 bildet mit dem Führungsrohr 30 eine Einheit, wobei von der Gesamtlänge Formkörper 610 und Führungsrohr 30 die Länge des Formkörpers 610 etwa der halben Gesamtlänge entspricht.

Dieser im oberen Bereich als Führungsrohr 30 ausgebildete Formkörper 610 ist mit einer obenseitigen Bohrung 611 versehen. Im Bereich der Innenwandfläche 611a der Bohrung ist mindestens eine Ringnut 612 ausgebildet, die radial verlaufend ist und z.B. einen dreieckförmigen Querschnitt aufweist, während der Implantatpfosten 211 an dem Außenumfang seines bodenseitigen, als Zapfen 211a ausgebildeten Endes eine der Anzahl der Ringnuten 612 in der Bohrung 611 des Formkörpers 610 entsprechende Anzahl von in die Ringnuten 612 bei in die Bohrung 611 des Formkörpers 610 eingesetzten Implantatpfosten 211 eingreifenden, wulstartig ausgebildeten Ringen 613 aufweist. Die Anzahl der Ringnuten 612 in der bohrung 611 des Formkörpers 610 kann beliebig gewählt sein ; für einen festen Sitz des Implantatpfostens 211 in dem Primärzylinder 10 ist bereits eine Ringnut 612 ausreichend (Fig.23, 24 und 25).

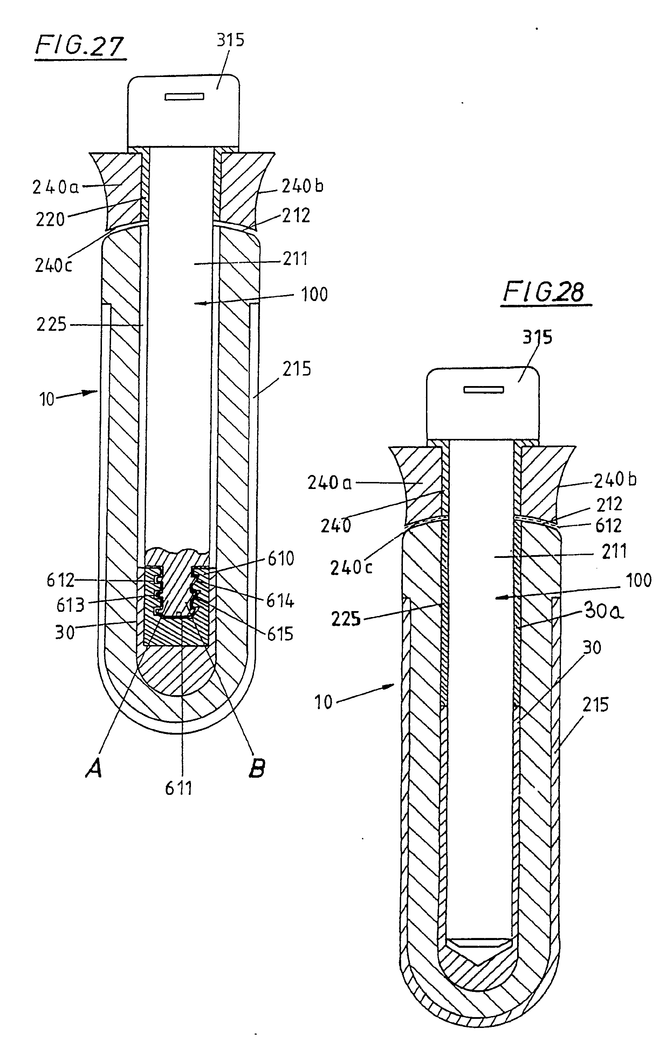

In gleicher Weise wie der Implantatpfosten 211 des Sekundärzylinders 100 in dem Primärzylinder 10 unter Verwendung des Formkörpers 610 gehalten ist, kann auch der Montagekopf 330 auf dem freien oberen Ende des Implantatpfostens 211 befestigt sein, der mit seinem oberen Ende mit einem Abschnitt aus dem Implantataufsatz 240 herausgeführt ist. Der Klemmverschluß für die Montagekappe 330 ist mit 640a bezeichnet und entsprechend dem Klemmverschluß 640 ausgebildet (Fig.23). In der Montagekappe 330 ist eine Bohrung 611b vorgesehen, die innenwandseitig eine Ringnut 612a aufweist (Fig. 26). Das obere freie Ende des Implantatpfostens 211 ist so ausgebildet, daß es in die Bohrung 611b einführbar ist. Ferner trägt der Implantatpfosten 211 an seinem oberen Außenumfang eine ringartige Wulst 613a, die so profiliert ist, daß sie nach dem Aufsetzen der Montagekappe 330 auf den Implantatpfosten 211 in die Ringnut 612a in den Montagekopf 330 eingreift (Fig. 25). Durch Einfügen der einzelnen Teile unter Anwendung eines leichten Druckes werden die Klemmverschlüsse 640, 640a gebildet (Fig. 23).

In Fig. 27 sind zwei verschiedene Ausgestaltungen eines Klemmverschlusses A und B dargestellt. Bei der Ausführungsform A sind konzentrische Ringnuten 612 vorgesehen, während der Implantatpfosten 211 konzentrische, wulstartig ausgebildete Ringe 613 auf seinem Außenumfang trägt, die so ausgebildet und angeordnet sind, daß nach dem Einsetzen des Implantatpfostens 211 in den Primärzylinder 10 diese Ringe 613 in die Ringnuten 612 eingreifen.

Bei der bei B angedeuteten Ausführungsform weist die Ringnut 612 bzw. die Ringnuten der Bohrung 611 des Formkörpers 610 zur Ausbildung oberer Hinterschneidungen 614 einen etwa dreieckförmigen Querschnitt auf, während der Implantatpfosten 211 an seinem bodenseitigen Außenumfang eine der Anzahl der Ringnuten 612 entsprechende Anzahl von ringartigen Eingriffprofilierungen 615 trägt, so daß nach dem Einsetzen des Implantatpfostens 211 mit seinem bodenseitigen Ende in die Bohrung 611 des Formkörpers 610 diese Eingriffprofilierungen 615 in die Ringnuten 612 einrasten, so daß aufgrund der beschriebenen und dargestellten konstruktiven Ausgestaltung ein Lösen des Klemmverschlusses nicht möglich ist.

Die Spaltdichtigkeit zwischen den beiden sphärischen Gleitflächen 212, 240c des Primärzylinders 10 und des Implantataufsatzes 240, 240a wird durch die Paßgenauigkeit und die formschlüssige Auflage der sphärischen Gleitflächen aufeinander zwischen dem Primärzylinder 10 und dem Implantataufsatz bzw. der Schleimhautmanschette, die den Keramikoberteil darstellt, und der Chemisorption erreicht, die durch den verwendeten keramischen Werkstoff bewirkt wird, nämlich insofern, als der verwendete keramische Werkstoff Sauerstoffionen an seiner Oberfläche freigibt, die sich mit dem umgebenden Milieu zu einer Gleitschicht verbinden, wobei für das Zustandekommen der Chemisorption normale Luftfeuchtigkeit ausreicht. Es wird auf diese Weise aufgrund der Ausnutzung der Materialeigenschaft ein Gleitfilm geschaffen.

Es besteht jedoch auch die Möglichkeit, die Spaltdichtigkeit mit einer Beschichtung 620 oder mit einer Einlage aus einem Polytetrafluoräthylen, das unter dem Handelsnamen "Teflon" bekannt ist, oder einem anderen geeigneten, insbesondere sauerstoffionendurchlässigen Werkstoff zu erreichen, wie dies in Fig. 28 dargestellt ist. Im wesentlichen kommen hierbei als Beschichtungsmaterialien solche Werkstoffe infrage, die in keiner Weise die Chemisorption beeinträchtigen. Anstelle einer Beschichtung können auch Formkörper in Form von Einlagen aus geeigneten Werkstoffen verwendet werden. Die Beschichtung aus den geeigneten Werkstoffen kann auf einer der beiden sphärischen Gleitflächen 212 bzw. 240c angebracht sein.

Wie Fig. 28 zeigt, kann der sonst vorgesehene Luftspalt 225 zwischen dem Führungsrohr 30 und dem oberen Modulelement 220 durch ein oberhalb des Führungsrohres 30 aufgesetztes weiteres rohrförmiges Modulelement 30a aus dauerelastischem Material, wie Kunststoff oder einem anderen geeigneten Werkstoff, ausgefüllt sein. Dieses zusätzliche Modulelement 30a gewährleistet eine ausreichende Spaltdichtigkeit.

Der obere umlaufende Rand des Modulelementes 220 ist manschettenartig nach außen abgewinkelt. Dieser abgewinkelte Abschnitt ist mit 220c bezeichnet und dient zur Kompensation der Stabstauchung bei auftretenden Vertikalkräften (Fig.23 und 25).

Der Implantatpfosten 211 kann aus einem vielkristallinen Werkstoff, z.B. einem Metall, mit einem hohen Elastizitätsmodul bestehen. Es hat sich gezeigt, daß die plastischen Eigenschaften des verwendeten metallischen Werkstoffes mit Ein- oder Vielkristallstruktur durch die Faktoren, die die Abweichungen des realen Gitters vom idealen Gitter verursachen, bestimmt werden. Hierbei handelt es sich insbesondere um die verschiedenen Arten von Gitterfehlstellen, die teils durch das Kristallwachstum bedingt sind, teils aber auch erst durch äußere Einwirkungen, z.B. der Herstellung und Bearbeitung des Metalles, gebildet werden. Jeder Gitterfehler ist eine Komponente für das plastische Verhalten eines Werkstoffes unter der Wirkung von Kräften, die weit unterhalb der theoretischen Schubfestigkeit eines sogenannten Einkristalles liegen. Plastizität zeigen die meisten Metalle bzw. Kristalle. Wirken äußere Kräfte auf diese metallischen Körper, z.B. auf den Implantatpfosten, so ändern sie ihre Gestalt vor dem Eintritt des Bruches, bleibend, im Gegensatz zu den spröden Werkstoffen, bei denen nach Überschreiten einer bestimmten Spannungsgrenze der Bruch eintritt.

Wird also bei einem plastisch verformten Körper die auf ihn wirkende Kraft weggenommen, so gehen die Verformungen nur zu einem geringen Teil zurück; zum größeren Teil bleiben sie als sogenannte Formänderungen bestehen, während elastisch verformte Körper, z.B. der Implantatpfosten, beim Wegnehmen der Kraft die Verformung zurückgeben. Der Implantatpfosten besteht deshalb aus einem spröden Werkstoff. Die Stärke des Implantatpfostens ist auf eine bestimmte Spannungsgrenze ausgerichtet, wobei die Kaumuskelkraft als äußere Kraft angenommen wird. Bedingt durch den Querschnitt und die Länge des Implantatpfostens wird ein Überschreiten einer bestimmten Spannungsgrenze und somit der Bruch des Implantatpfostens verhindert, der unter diesen Bedingungen als Körper bei gleichmäßiger Bewegung um seine Ruhelage schwingt.

Aufgrund der Ausgestaltung, daß der Implantatpfosten 211 im Bereich des Implantataufsatzes 240, 240a von einem Modulelement 220 aus einem stark elastischen Werkstoff umgeben ist, daß im bodenseitigen Bereich der Implantatpfosten in einem Führungsrohr 30 aus einem elastischen Werkstoff gehalten ist, der gegenüber der Elastizität des Modulelementes 220 eine geringere Elastizität aufweist, und daß zwischen dem Modulelement 220 und dem Führungsrohr 30 ein modulelementfreier Abschnitt ausgebildet ist, wird der Hebeldrehpunkt des Implantatpfostens 211 in das untere Drittel des Primärzylinders 10 verlegt. Das Modulelement 220 besteht zur Erzielung seiner Elastizität aus einem die jeweils erforderliche Elastizität aufweisenden Werkstoff, wie aus einem Kunststoff, z.B. Silikon-Kautschuk ; jedoch auch andere geeignete Werkstoffe können zur Anwendung gelangen.

In den Fig. 6 und 14 sind mit S der Schwingungsbereich und mit L der Lagerbereich bezeichnet. Der Implantatpfosten 211 ist im Lagerbereich L sicher in dem Führungsrohr 30 gehalten, so daß hier eine großflächige Energieübertragung der nicht schon im Schwingungsbereich S in Wärme verwandelten Energie auf den Primärzylinder 10 ermöglicht wird.

## Ansprüche

1. Enossales Implantat zur Befestigung von festsitzendem oder abnehmbarem Zahnersatz, bestehend aus zwei miteinander verbindbaren Teilen, von denen das eine Teil als in den Kieferknochen einbringbarer und sich in diesem kraftschlüssig verankernder Primärzylinder (10) mit einer mittigen Längsbohrung (13) und das andere Teil als Sekundärzylinder (100) ausgebildet ist, der einen in die Längsbohrung (13) des Primärzylinders einbringbaren und in diesem gehaltenen Implantatpfosten (211) zur Aufnahme des Zahnersatzes aufweist, und aus einem an dem Implantatpfosten (211) vorgesehenen Implantataufsatz (240), wobei der Implantatpfosten (211) an seinem freien oberen Ende für den Anschluß des Zahnersatzes ausgebildet ist und von einem die im Bereich des Zahnersatzes oder im Munde auftretenden Horizontal- und/oder Vertikal- und/oder Torsions-Kräfte und Schwingungen in den Sekundärzylinder (100) und von dort in den Primärzylinder (10) umleitenden Kraftleitungssystem aus mehreren, sich aneinanderreihenden Bereichen mit unterschiedlichen elastischen Eigenschaften umgeben und in dem Primärzylinder (10) fest oder lösbar gehalten ist, dadurch gekennzeichnet, daß der Implantatpfosten (211) als einarmiger Hebel in der Längsbohrung (13) des Primärzylinders (10) angeordnet ist, daß zur Umleitung der im Bereich des Zahnersatzes oder im Munde auftretenden Horizontal- und/oder Vertikal- und/oder Torsions-Kräfte und Schwingungen in den bodenseitigen Bereich des Sekundärzylinders (100) und von dort in den Primärzylinder (10) oder in den Boden des Primärzylinders (10) das Kraftleitungssystem zur Verlagerung des Hebeldrehpunktes des Implantatpfostens (211) in den unteren Bereich des Primärzylinders (10) aus einem den Implantatpfosten (211) im Bereich des Implantataufsatzes (240 ; 240a) umgebenden Modulelement (220) aus einem stark elastischen Werkstoff besteht, wobei der Implantataufsatz (240 ; 240a) mit einer mit der Längsbohrung (13) des Primärzylinders (10) fluchtenden, das Modulelement (220) aufnehmenden Durchbohrung (241) versehen und auf der Oberfläche des Primärzylinders (10) gleitend gehalten ist, und daß der Implantatpfosten (211) durch einen an dem Implantatpfosten bei eingesetztem Sekundärzylinder (100) angeordneten Thermoverschluß, fest mittels einer Klebeverbindung oder mittels lösbarer oder unlösbarer Klemmverschlüsse mit dem Primärzylinder (10) verbunden ist.

2. Enossales Implantat nach Anspruch 1, dadurch gekennzeichnet, daß das Kraftleitungssystem aus dem den Implantatpfosten (211) im Bereich des Implantataufsatzes (240 ; 240a) umgebenden Modulelement (220) aus einem stark elastischen Werkstoff, aus einem sich von der Längsbohrungseintrittsöffnung (211a) erstreckenden, einen Luftspalt (225) bildenden, modulelementfreien Abschnitt und aus einem in einem Abstand von der Längsbohrungseintrittsöffnung (211a) angeordneten und sich an den modulelementfreien Abschnitt anschließenden Führungsrohr (30) aus einem elastischen Werkstoff besteht, das in dem Primärzylinder (10) mittels einer Klebeverbindung gehalten ist und daß der Implantatpfosten (211) in dem Führungsrohr (30) durch einen an dem Implantatpfosten (211) bei eingesetztem Sekundärzylinder (100) im Bereich des Führungsrohres (30) angeordneten Thermoverschluß, fest mittels einer Klebeverbindung oder mittels lösbarer oder unlösbarer Klemmverschlüsse mit dem Primärzylinder (10) verbunden ist.

3. Enossales Implantat nach einem der Ansprüche 1 und 2, dadurch gekennzeichnet, daß das obere Modulelement (220) im Innenraum der Durchbohrung (241) des auf dem Primärzylinder (10) gleitend gehaltenen Implantataufsatzes (240 ; 240a) angeordnet und mittels einer Klebeverbindung an dem Implantataufsatz (240 ; 240a) gehalten ist.

4. Enossales Implantat nach einem der Ansprüche 1 bis 3, dadurch gekennzeichnet, daß das obere Modulelement (220) an seinem oberen Endbereich (220a) einen auskragenden, scheibenförmigen Abschnitt (220b) und im Bereich des Abschnittes (220b) mittels einer Klebeverbindung an dem Implantataufsatz (240 ; 240a) und/oder an dem Kopf (315) des Implantatpfostens (211) gehalten ist.

5. Enossales Implantat nach einem der Ansprüche 1 bis 4, dadurch gekennzeichnet, daß der Thermoverschluß aus einem Draht aus Bimetall, z.B. Memorymetall, besteht, der in einer an dem Implantatpfosten (211) angeordneten Ringnut gehalten ist.

6. Enossales Implantat nach einem der Ansprüche 1 bis 5, dadurch gekennzeichnet, daß der Thermoverschluß aus einem oder mehreren Drähten aus Bimetall, z.B. Memorymetall, besteht, die in in den Innenraum einer Längsdurchbohrung (311a) des Implantatpfostens (211) hineinreichenden Längsdurchbrechungen (313a, 313b, 313c, 313d) angeordnet sind.

7. Enossales Implantat nach einem der Ansprüche 1 bis 6, dadurch gekennzeichnet, daß die Gleitfläche (212) des Primärzylinders (10) für den Implantataufsatz (240 ; 240a) als Kalotte mit einem Winkel $\alpha$ von insbesondere 9,27° ausgebildet ist.

8. Enossales Implantat nach einem der Ansprüche 1 bis 7, dadurch gekennzeichnet, daß der Implantataufsatz (240a) als etwa zylindrischer Formkörper ausgebildet ist, dessen Außenwandfläche (240b) kreisbogenförmig eingezogen ist und dessen unterer Außendurchmesser etwa dem Außendurchmesser des Primärzylinders (10) entspricht, wobei der obere Außendurchmesser des Implantataufsatzes (240a) gegenüber dem unteren Außendurchmesser gleich oder größer bemessen ist, und daß die untere Auflagefläche (240c) der Form der Gleitfläche (212) des Primärzylinders (10) entsprechend ausgebildet und sich dieser anpassend ist.

9. Enossales Implantat nach Anspruch 1 bis 8, dadurch gekennzeichnet, daß die der Kortikalis entsprechende Kontaktzone (A) des Primärzylinders (10) außen beschichtungsfrei ist, während die der Spongiosa entsprechende Kontaktzone (B) des Primärzylinders (10) die Außenbeschichtung (215) trägt.

10. Enossales Implantat nach Anspruch 1 bis 9, dadurch gekennzeichnet, daß der Implantatpfosten (211) des Sekundärzylinders (100) insbesondere in seinem unteren Bereich mit dem Führungsrohr (30) mittels einer Klebeverbindung fest verbunden ist.

11. Enossales Implantat nach Anspruch 1 bis 10, durch gekennzeichnet, daß der Primärzylinder (10) eine Außenbeschichtung (215) insbesondere aus Hydroxylapatitkeramik aufweist.

12. Enossales Implantat nach Anspruch 1 bis 11, dadurch gekennzeichnet, daß der Implantatpfosten (211) führungshülsenfrei mit seinem bodenseitigen Endbereich fest mit der Wandung der Längsbohrung (13) des Primärzylinders (10) mittels einer Klebeverbindung verbunden ist.

13. Enossales Implantat nach einem der vorangegangenen Ansprüche 1 bis 12, dadurch gekennzeichnet, daß der Implantatpfosten (211) führungshülsenfrei mittels einer Schraubverbindung (536) oder einer anderen geeigneten, gleichwertigen Verbindung mit einem im Innenraum des Primärzylinders (10) mittels einer Klebeverbindung (537) gehaltenen, den gesamten, sonst das Führungsrohr aufnehmenden Raum einschließlich des darunter befindlichen Hohlraumes zwischen dem bodenseitigen Ende des sonst vorgesehenen Führungsrohres (30) und dem Primärzylinderboden ausfüllenden Formkörper (535) mit einer oberen Bohrung für die Aufnahme des Implantatpfostens (211) verbunden ist.

14. Enossales Implantat nach Anspruch 1 bis 13, dadurch gekennzeichnet, daß der Primärzylinder (10) aus Keramik, Titan oder anderen geeigneten Werkstoffen besteht.

15. Enossales Implantat nach Anspruch 1 bis 14, dadurch gekennzeichnet, daß der Implantatpfosten (211) des Sekundärzylinders (100) in dem Primärzylinder (10) mittels eines Klemmverschlusses (630) gehalten ist, der aus einem in der Längsbohrung (13) des Primärzylinders (10) angeordneten Formkörper (600) mit einer oberen Halterung (602) und aus einem am bodenseitigen Ende des Implantatpfostens (211) angeordneten, die Halterung (602) klemmend umgreifenden, kapselförmigen Element (603) aus einem Bimetall, insbesondere Memorymetall, besteht.

16. Enossales Implantat nach Anspruch 15, dadurch gekennzeichnet, daß das im bodenseitigen Bereich mittels einer Klebeverbindung in dem Primärzylinder (10) angeordnete Führungsrohr (30) eine kurze Länge aufweist und in seinem Innenraum einen als Vollzylinder ausgebildeten Formkörper (600) aus einem metallischen Werkstoff oder einem anderen geeigneten Werkstoff trägt, der mittels einer Klebeverbindung mit dem Führungsrohr (30) fest verbunden ist und eine der Länge des Führungsrohres (30) entsprechende Länge aufweist, und daß der Formkörper (600) auf seiner dem Modulelement (220) zugekehrten Seite (601) eine kugelförmige oder eine andere geeignete geometrische Form aufweisende Halterung (602) trägt, die von einem kapselförmigen Element (603) umgriffen wird, das am bodenseitigen Ende des Implantatpfostens (211) an diesem angeformt ist und dessen der Halterung (602) zugekehrte Wandfläche (603a) einen Bimetall-, z.B.Memorymetall-, Streifen (604) oder eine Innenbeschichtung, Einlage od.dgl. aus einem elastischen Kunststoff oder einem anderen geeigneten elastischen Werkstoff trägt.

17. Enossales Implantat nach Anspruch 16, dadurch gekennzeichnet, daß im bodenseitigen Bereich der Längsbohrung (13) in dieser mittels einer Klebeverbindung ein Formkörper (600) angeordnet ist, der obenseitig eine kugelförmige oder eine andere geeignete geometrische Form aufweisende Halterung (602) aufweist, die von einem kapselförmigen Element (603) umgriffen wird, das am bodenseitigen Ende des Implantatpfostens (211) befestigt ist und aus einem Bimetall, insbesondere Memorymetall, besteht.

18. Enossales Implantat nach Anspruch 1 bis 17, dadurch gekennzeichnet, daß der Montagekopf (330) mit einem dem Klemmverschluß (630) entsprechend ausgebildeten Klemmverschluß (630a) auf dem Implantatpfosten (211) des Sekundärzylinders (100) befestigt ist, wobei der Implantatpfosten (211) an seinem oberen Ende mit einer kugelförmigen oder eine andere geeignete geometrische Form aufweisenden Halterung (602a) versehen ist und der Montagekopf (330) in seinem Innenraum ein klauenförmiges, die Halterung (602a) umgreifendes Element (603a) aufweist, das aus Bimetall, insbesondere Memorymetall, besteht.

19. Enossales Implantat nach Anspruch 1 bis 18, dadurch gekennzeichnet, daß der Implantatpfosten (211) des Sekundärzylinders (100) in dem Primärzylinder (10) mittels eines Klemmverschlusses (640) gehalten ist, der aus einem in der Längsbohrung (13) des Primärzylinders (10) angeordneten Formkörper (610) mit einer mittigen mit einer inneren Ringnut (612) versehenen Bohrung (611) und aus einer an dem bodenseitigen Ende des Implantatpfostens (211) an dessen Außenumfang angeformten, bei in dem Primärzylinder (10) eingesetztem Implantatpfosten (211) in die Ringnut (612) eingreifenden oder einrastenden Ringwulst (613) besteht.

20. Enossales Implantat nach Anspruch 19, dadurch gekennzeichnet, daß die Ringnut (612) bzw. Ringnuten der Bohrung (611) des Formkörpers (610)

zur Ausbildung oberer Hinterschneidungen (614) einen Querschnitt aufweist, der einem rechtwinkligen Dreieck entspricht und daß der Implantatpfosten (211) an seinem bodenseitigen Außenumfang eine der Anzahl der Ringnuten (612) entsprechende Anzahl von ringartigen Eingriffsprofilierungen (615) zur Ausbildung eines unlösbaren Klemmverschlusses trägt.

21. Enossales Implantat nach Anspruch 19, dadurch gekennzeichnet, daß der Formkörper (610) vollwandig ausgebildet ist und an seiner dem Modulelement (220) zugekehrten Seite ein Führungsrohr (30) trägt, daß der Formkörper (610) und das Führungsrohr (30) mittels einer Klebeverbindung mit der Innenwandfläche der Längsbohrung (13) fest verbunden ist, daß der Formkörper (610) etwa gleich lang bemessen wie das Führungsrohr (30) ist, daß zwischen dem Führungsrohr (30) und dem Modulelement (220) ein Luftspalt (225) ausgebildet ist, daß der Formkörper (610) mit einer oberen Bohrung (611) versehen ist, in deren Innenwandfläche (611a) mindestens eine Ringnut (612) ausgebildet ist, und daß der Implantatpfosten (211) an seinem unteren Ende zapfenartig ausgebildet ist und an seinem Außenumfang eine der Anzahl der Ringnuten (612) entsprechende Anzahl von in die Ringnuten (612) einrastbaren Ringwülsten (613) aufweist, wobei der Innendurchmesser der Bohrung (611) in dem Formkörper (610) gegenüber dem Außendurchmesser des Implantatpfostens (211) kleiner bemessen ist und der Außendurchmesser des Implantatpfostens dem Innendurchmesser der Bohrung (611) entspricht.

22. Enossales Implantat nach Anspruch 19 bis 21, dadurch gekennzeichnet, daß der Montagekopf (330) mit einem dem Klemmverschluß (640) entsprechend ausgebildeten Klemmverschluß (640a) auf dem Implantatpfosten (211) des Sekundärzylinders (100) befestigt ist, wobei der Implantatpfosten (211) am Außenumfang seines oberen freien Endes eine Ringwulst (613a) trägt, die in eine Ringnut (612a) in der Innenwandfläche einer in dem Montagekopf (330) ausgebildeten Bohrung (611b) einrastet.

23. Enossales Implantat nach Anspruch 1 bis 22, dadurch gekennzeichnet, daß zur Ausbildung einer Spaltdichtigkeit zwischen den beiden sphärischen Gleitflächen (212, 240c) des Primärzylinders (10) und des Implantataufsatzes (240 ; 240a) mindestens eine der beiden Gleitflächen mit einer Beschichtung (620) oder mit einer Einlage aus einem Polytetrafluoräthylen oder einem anderen geeigneten, insbesondere sauerstoffionendurchlässigen Werkstoff versehen ist.

24. Enossales Implantat nach Anspruch 1 bis 23, dadurch gekennzeichnet, daß der Luftspalt (225) zwischen dem Führungsrohr (30) und dem oberen Modulelement (220) durch ein oberhalb des Führungsrohres (30) aufgesetztes, rohrförmiges Modulelement (30a) aus dauerelastischem Material ausgefüllt ist.

25. Enossales Implantat nach Anspruch 1 bis 24, dadurch gekennzeichnet, daß der obere umlaufende Rand des Modulelementes (220) manschettenartig nach außen abgewinkelt ist.

## Revendications

1. Implant endo-osseux pour fixer des dents artificielles étant arrêtées ou amovibles, comportant deux parties pouvant être reliées l'une à l'autre, l'une de ces parties étant configurée comme un cylindre primaire (10), avec un alésage longitudinal central (13), pouvant être mis en place dans le maxillaire et s'ancrant dans celui-ci par adhérence et l'autre partie étant configurée comme un cylindre secondaire (100) qui présente un montant d'implant (211) pour loger les dents artificielles qui peut être mis en place dans l'alésage longitudinal (13) du cylindre primaire et qui est maintenu dans celui-ci, et comportant en outre une pièce rapportée sur l'implant (240) prévue sur le montant d'implant (211), le montant d'implant (211) étant formé, à son extrémité supérieure libre, pour le raccord des dents artificielles et étant entouré par un système de guidage de forces qui fait dériver les forces horizontales et/ou verticales et/ou de torsion et les vibrations, qui interviennent dans la zone des dents artificielles ou dans la bouche, dans le cylindre secondaire (100) et de là dans le cylindre primaire (100), système qui se compose de plusieurs zones à la file l'une de l'autre, avec différentes propriétés élastiques, et étant maintenu dans le cylindre primaire (10) de manière fixe ou amovible, caractérisé en ce que le montant de l'implant (211) est placé comme un levier à bras unique dans l'alésage longitudinal (13) du cylindre primaire (10), que, pour dériver les forces horizontales et/ou verticales et/ou de torsion et les vibrations, qui interviennent dans la zone des dents artificielles ou dans la bouche, dans la zone située du cylindre secondaire (100) située du côté du fond et de là dans le cylindre primaire (10) ou dans le fond du cylindre primaire (10), le système de guidage de forces pour décaler le point d'appui du levier du montant d'implant (211) dans la zone inférieure du cylindre primaire (10) est constitué par un élément modulaire (220) en une matière très élastique qui entoure le montant d'implant (211) dans la zone de la pièce rapportée de l'implant (240 ; 240a), la pièce rapportée de l'implant (240 ; 240a) étant pourvue d'un perçage (241), aligné sur l'alésage longitudinal (13) du cylindre primaire (10), qui loge l'élément modulaire (220) et étant maintenue coulissante sur la surface du cylindre primaire (10) et que le montant d'implant (211) est relié au cylindre primaire (10) de manière fixe au moyen d'un collage ou au moyen de fermetures par serrage amovibles ou inamovibles par une fermeture thermique placée sur le montant d'implant, lorsque le cylindre secondaire (100) est en place.

2. Implant endo-osseux selon la revendication 1, caractérisé en ce que le système de guidage de forces est constitué par l'élément modulaire (220) en une matière très élastique qui entoure le montant d'implant (211) dans la zone de la pièce rapportée de l'implant (240 ; 240a), par une portion exempte d'élément modulaire, qui s'étend à partir de l'orifice d'entrée de l'alésage longitudinal (211a) et qui forme une fente d'aération (225) et par une tube de guidage (30) en une matière élastique qui est placé à une certaine distance de l'orifice d'entrée de l'alésage longitudinal (211a) et qui fait suite à la portion exempte d'élément modulaire, tube de guidage qui est maintenu dans le cylindre primaire (10) au moyen d'un collage, et que le montant d'implant (211) est relié au cylindre primaire (10) de manière fixe au moyen d'un collage ou au moyen de fermetures par serrage amovibles ou inamovibles par une fermeture thermique placée sur le montant d'implant, lorsque le cylindre secondaire (100) est en place.

3. Implant endo-osseux selon l'une des revendications 1 et 2, caractérisé en ce que l'élément modulaire supérieur (220) est placé dans l'espace intérieur du perçage (241) de la pièce rapportée de l'implant (240 ; 240a) qui est maintenue coulissante sur le cylindre primaire (10) et qu'il est maintenu sur la pièce rapportée de l'implant (240 ; 240a) au moyen d'un collage.

4. Implant endo-osseux selon l'une des revendications 1 à 3, caractérisé en ce que l'élément modulaire supérieur (220) présente, dans la zone de son extrémité supérieure (220a) une portion en forme de disque (220b) qui fait saillie et qu'il est maintenu, dans la zone de la portion (220b), sur la pièce rapportée de l'implant (240 ; 240a) et/ou sur la tête (315) du montant de l'implant (211) par un collage.

5. Implant endo-osseux selon l'une des revendications 1 à 4, caractérisé en ce que la fermeture thermique est constituée par un fil en bimétal, par exemple en métal à mémoire, qui est maintenu dans une rainure annulaire placée sur le montant de l'implant (211).

6. Implant endo-osseux selon l'une des revendications 1 à 5, caractérisé en ce que la fermeture thermique est constituée par un ou plusieurs fils en bimétal, par exemple en métal à mémoire, qui sont placés dans des perçages longitudinaux (313a, 313b, 313c, 313d) qui s'étendent jusque dans l'espace intérieur d'un alésage longitudinal (311a) du montant d'implant (211).

7. Implant endo-osseux selon l'une des revendications 1 à 6, caractérisé en ce que la surface de glissement (212) du cylindre primaire (10) pour la pièce rapportée de l'implant (240 ; 240a) est configurée comme une calotte sphérique avec un angle $\alpha$ d'en particulier 9,27°.

8. Implant endo-osseux selon l'une des revendications 1 à 7, caractérisé en ce que la pièce rapportée de l'implant (240a) est configurée comme un corps moulé à peu près cylindrique dont la face de paroi extérieure (240b) est rentrée en arc de cercle et dont le diamètre extérieur inférieur correspond à peu près au diamètre extérieur du cylindre primaire (10), le diamètre extérieur supérieur de la pièce rapportée de l'implant (240a) étant dimensionné en étant égal ou supérieur au diamètre extérieur inférieur et que la surface d'appui inférieure (240c) est configurée de manière à correspondre à la forme de la surface de glissement (212) du cylindre primaire (10) et qu'elle s'adapte à celle-ci.

9. Implant endo-osseux selon les revendications 1 à 8, caractérisé en ce que la zone de contact (A) du cylindre primaire (10) qui correspond au cortex est exempte de revêtement à l'extérieur, tandis que la zone de contact (B) du cylindre primaire (10) qui correspond à la substance spongieuse porte le revêtement extérieur (215).

10. Implant endo-osseux selon les revendications 1 à 9, caractérisé en ce que le montant d'implant (211) du cylindre secondaire (100) est relié de manière fixe au tube de guidage (30) par un collage, en particulier dans sa zone inférieure.

11. Implant endo-osseux selon les revendications 1 à 10, caractérisé en ce que le cylindre primaire (10) présente un revêtement extérieur (215) en particulier en céramique d'hydroxylapatite.

12. Implant endo-osseux selon les revendications 1 à 11, caractérisé en ce que le montant d'implant (211) est relié par sa zone terminale située du côté du fond, sais douille de guidage, de manière fixe à la paroi de l'alésage longitudinal (13) du cylindre primaire (10) au moyen d'un collage.

13. Implant endo-osseux selon l'une des revendications précédentes 1 à 12, caractérisé en ce que le montant d'implant (211) est relié à un corps moulé (535) avec un alésage supérieur pour recevoir le montant de l'implant (211), sans douille de guidage, au moyen d'un raccord à vis (536) ou d'un autre raccord approprié équivalent, corps moulé qui est maintenu dans l'espace intérieur du cylindre primaire (10) au moyen d'un collage (537) et qui remplit tout l'espace, et sinon l'espace qui reçoit le tube de guidage, y compris l'espace creux qui se trouve en dessous entre l'extrémité du tube de guidage (30), qui est sinon prévu, qui est située du côté du fond et le fond du cylindre primaire.

14. Implant endo-osseux selon les revendications 1 à 13, caractérisé en ce que le cylindre primaire (10) est constitué par de la céramique, du titane ou d'autres matières appropriées.

15. Implant endo-osseux selon les revendications 1 à 14, caractérisé en ce que le montant d'implant (211) du cylindre secondaire (100) est maintenu dans le cylindre primaire (10) au moyen d'une fermeture ar serrage (630) qui est constituée par un corps moulé (600), avec une fixation supérieure (602), placé dans l'alésage longitudinal (13) du cylindre primaire (10) et par un élément en forme de capsule (603), constitué par un bimétal, en particulier un métal à mémoire, placé à l'extrémité du montant de l'implant (211) située du côté du fond et qui enveloppe la fixation (602) en la serrant.

16. Implant endo-osseux selon la revendication 15, caractérisé en ce que le tube de guidage (30) placé dans le cylindre primaire (10) dans la zone située du côté du fond au moyen d'un collage est de longueur courte et porte, dans son espace intérieur, un corps moulé (600) configuré comme un cylindre plein et constitué par une matière métallique ou par une autre matière appropriée, corps moulé qui est relié de manière fixe au tube de guidage (30) au moyen d'un collage et qui a une longueur qui correspond à la longueur du tube de guidage (30) et que le corps moulé (600) porte, sur sa face (601) tournée vers l'élément modulaire (220), une fixation (602) qui présente une forme sphérique ou une autre forme géométrique apropriée, fixation qui est enveloppée par un élément en forme de capsule (603) qui est moulé sur le montant d'implant (211) à son extrémité située du côté du fond et dont la face de paroi (603a) tournée vers la fixation (602) porte une bande de bimétal (604), par exemple de métal à mémoire, ou un revêtement intérieur, une garniture intérieure ou équivalent en une matière plastique élastique ou en une autre matière élastique appropriée.

17. Implant endo-osseux selon la revendication 16, caractérisé en ce qu'un corps moulé (600) est placé, dans la zone de l'alésage longitudinal (13) située du côté du fond, dans cet alésage au moyen d'un collage, corps moulé qui présente une fixation (602) qui présente, sur sa face supérieure, une forme sphérique ou une autre forme géométrique appropriée et qui est enveloppée par un élément en forme de capsule (603) qui est fixé à l'extrémité du montant d'implant (211) située du côté du fond et constitué par un bimétal, en particulier par du métal à mémoire.

18. Implant endo-osseux selon les revendications 1 à 17, caractérisé en ce que la tête de montage (330) est fixée sur le montant d'implant (211) du cylindre secondaire (100) avec une fermeture par serrage (630a) configurée de manière correspondante à la fermeture par serrage (630), le montant d'implant (211) étant pourvu à son extrémité supérieure d'une fixation (602a) sphérique ou présentant une autre forme géométrique appropriée et que la tête de montage (330) présente, dans son espace intérieur, un élément (603a) en forme de griffe qui enveloppe la fixation (602a) et qui est constitué par un bimétal, en particulier un métal à mémoire.

19. Implant endo-osseux selon les revendications 1 à 18, caractérisé en ce que le montant d'implant (211) du cylindre secondaire (100) est maintenu dans le cylindre primaire (10) au moyen d'une fermeture par serrage (640) qui est constituée par un corps moulé (610) avec un alésage central (611) pourvu d'une rainure annulaire intérieure (612), corps moulé qui est placé dans l'alésage longitudinal (13) du cylindre primaire (10) et par un bourrelet annulaire (613), moulé à l'extrémité du montant d'implant (211) située du côté du fond sur la périphérie extérieure du montant d'implant, qui s'engrène ou s'enclenche dans la rainure annulaire (612) lorsque le montant d'implant (211) est en place dans le cylindre primaire (10).

20. Implant endo-osseux selon la revendication 18, caractérisé en ce que la rainure annulaire (612) ou les rainures annulaires de l'alésage (611) du corps moulé (610) présente une section qui correspond à un triangle rectangle pour former des contre-dépouilles supérieures et que le montant d'implant (211) porte, sur sa périphérie extérieure du côté du fond, un nombre de profilages d'engrènement (615) de type annulaire correspondant au nombre des rainures annulaires (612) pour former une fermeture par serrage inamovible.

21. Implant endo-osseux selon la revendication 19, caractérisé en ce que le corps moulé (610) est configuré avec des parois pleines et qu'il porte un tube de guidage (30) sur sa face tournée vers l'élément modulaire (220), que le corps moulé (610) avec le tube de guidage (30) est relié de manière fixe à la face de paroi intérieure de l'alésage longitudinal (13) au moyen d'un collage, que le corps moulé (610) est dimensionné en étant à peu près aussi long que le tube de guidage (30), qu'une fente d'aération (225) est formée entre le tube de guidage (30) et l'élément modulaire (220), que le corps moulé (610) est pourvu d'un alésage supérieur (611) dans la face de paroi intérieure duquel au moins une rainure annulaire (612) est formée et que le montant d'implant (211) est formé en forme de tourillon à son extrémité inférieure et qu'il présente, sur sa périphérie extérieure, un nombre de bourrelets annulaires (613) qui peuvent s'enclencher dans les rainures annulaires (612) qui correspond au nombre des rainures ainulaires, le diamètre intérieur de l'alésage (611) dans le corps moulé (610) étant dimensionné en étant inférieur au diamètre extérieur du montant d'implant (211) et le diamètre extérieur du montant d'implant correspondant au diamètre intérieur de l'alésage (611).

22. Implant endo-osseux selon les revendications 19 à 21, caractérisé en ce que la tête de montage (330) est fixée sur le montant d'implant (211) du cylindre secondaire (100) avec une fermeture par serrage (640a) configurée de manière correspondante à la fermeture par serrage (640), le montant d'implant (211) portant, sur la périphérie extérieure de son extrémité libre supérieure, un bourrelet annulaire (613a) qui s'enclenche dans une rainure annulaire (612a) de la face de paroi intérieure d'un alésage (611b) configuré dans la tête de montage (330).

23. Implant endo-osseux selon les revendications 1 à 22, caractérisé en ce que, pour former une étanchéité de fente entre les deux surfaces de glissement sphériques (212, 240c) du cylindre primaire (10) et de la pièce rapportée d'implant (240 ; 240a), au moins l'une des deux surfaces de glissement est pourvue d'un revêtement (620) ou d'une garniture intérieure en un polytétrafluoréthylène ou en une autre matière appropriée, en particulier une matière perméable aux ions d'oxygène.

24. Implant endo-osseux selon les revendications 1 à 23, caractérisé en ce que la fente d'aération (225) entre le tube de guidage (30) et l'élément modulaire supérieur (220) est remplie par un élément modulaire en forme de tube (30a), en matière à élasticité permanente, placé au-dessus du tube de guidage (30).

25. Implant endo-osseux selon les revendications 1 à 24, caractérisé en ce que le bord périmétrique supérieur de l'élément modulaire (220) est coudé vers l'extérieur à la façon d'une manchette.

## Claims

1. Endosseous implant for fixing tight or removable dental prosthesis, comprising two interconnectable portions, of which the one portion is constructed in the form of a primary cylinder (10) to be inserted into the jawbone and nonpositively secured therein with a central longitudinal bore (13) and the other portion in the form of a secondary cylinder (100), which is provided with an implant stud (211) for receiving the dental prosthesis to be introduced into the longitudinal bore (13) of the primary cylinder and retained in the latter, and an implant attachment section (240) provided on the implant stud (211), wherein the implant stud (211), on its free upper end, is constructed so as to connect with the dental prosthesis and is surrounded by a force transmission system which diverts the horizontal and/or vertical and/or torsional forces and vibrations which occur within the area of the dental prosthesis or in the mouth into the secondary cylinder (100) and, from there, into the primary cylinder (10) from several areas in side-by-side arrangement which possess different elastic properties and is retained rigidly or detachably in the primary cylinder, characterized in that the implant stud (211) is disposed in the form of a one-armed lever in the longitudinal bore (13) of the primary cylinder (10), in that, in order to divert the horizontal and/or vertical and/or torsional forces and vibrations which occur within the area of the dental prosthesis or in the mouth into the bottom area of the secondary cylinder (100) and, from there, into the primary cylinder (10) or into the bottom of the primary cylinder (10), the force transmission system, for the purpose of transferring the lever fulcrum of the implant stud (211) into the lower region of the primary cylinder (10) from a modular element (220) surrounding the implant stud (211) within the area of the implant attachment section (240 ; 240a) fabricated from a highly resilient material, in which the implant attachment section (240 ; 240a) is provided with a bore (241) which is aligned with the longitudinal bore (13) of the primary cylinder for receiving the modular element (220) and is glidingly retained on the surface of the primary cylinder (10), and in that the implant stud (211), by means of a thermal closure mounted on the implant stud (211) with the secondary cylinder (100) in the inserted state, is, with the aid of an adhesive joint or by means of detachable or nondetachable clamping closures, firmly secured to the primary cylinder (10).

2. Endosseous implant according to Claim 1, characterized in that the force transmission system of the modular element (220) fabricated from a highly resilient material surrounding the implant stud (211) within the area of the implant attachment section (240 ; 240a), comprises a section devoid of any modular element which extends from the aperture of the longitudinal bore (211a) and forms an air gap (225) and a guide tube (30) fabricated from a resilient material which is disposed at a distance from the longitudinal bore aperture (211a) and follows the section which is devoid of any modular element, said tube being retained with the aid of a bonded joint, and in that the implant stud (211), by means of a thermal closure mounted on the implant stud (211) when the secondary cylinder (100) is inserted within the area of the guide tube (30), is securely retained in the guide tube (30) by means of an adhesive joint or with the aid of detachable or nondetachable clamping closures, is connected to the primary cylinder (10).

3. Endosseous implant according to either Claim 1 or 2, characterized in that the upper modular element (220) is disposed within the interior of the bore (241) of the implant attachment section (240 ; 240a) retained glidingly on the primary cylinder (10) and, with the aid of an adhesive joint, retained on the implant attachment section (240 ; 240a).

4. Endosseous implant according to any of Claims 1 thru 3, characterized in that the upper modular element (220), on its top terminal zone (220a), is provided with a projecting disk-like section (220b) and, within the area of the section (220b), by means of an adhesive joint, is retained on the implant attachment section (240 ; 240a) and/or on the head (315) of the implant stud (211).

5. Endosseous implant according to any of Claims 1 thru 4, characterized in that the thermal closure comprises a bi-metallic wire, e.g. of memorious metal, which is retained in an annular groove constructed on the implant stud (211).

6. Endosseous implant according to any of Claims 1 thru 5, characterized in that the thermal closure comprises one or several wires, e.g : of memorious metal, which are disposed within the longitudinal perforations (313a, 313b, 313c, 313d) extending into the interior of a longitudinal bore (311a) of the implant stud (211).

7. Endosseous implant according to any of Claims 1 thru 6, characterized in that the gliding area (212) of the primary cylinder (10) of the implant attachment section (240 ; 240a) is constructed with a cap-shaped configuration having an angle α of, in particular, 9.27°.

8. Endosseous implant according to any of Claims 1 thru 7, characterized in that the implant attachment section (240a) is constructed in the form of an approximately cylindrical shaped member, the outer wall area of which (240b) is recessed in an arcuate fashion and the lower external diameter of which corresponds approximately to the outer diameter of the primary cylinder (10), wherein the upper external diameter of the implant attachment section (240a), in comparison with the lower outer diameter, is dimensioned so as to be equal or larger, and in that the lower bearing surface (240a) is constructed so as to conform to the configuration of the gliding area (212) of the primary cylinder (10) and so as to adapt to the latter.

9. Endosseous implant according to any of Claims 1 thru 8, characterized in that the contact zone (A) corresponding to the corticalis of the primary cylinder (10) is, externally, devoid of any coating, while the contact area (B) corresponding to the spongiosa of the primary cylinder (10) bears the external coating (215).

10. Endosseous implant according to any of Claims 1 thru 9, characterized in that the implant stud (211) of the secondary cylinder (100), in particular within its lower area, is rigidly connected to the guide tube (30) by means of an adhesive joint.

11. Endosseous implant according to any of Claims 1 thru 10, characterized in that the primary cylinder (10) is provided with an external coating (215) more particularly of hydroxyl apatite ceramic material.

12. Endosseous implant according to any of Claims 1 thru 10, characterized in that the implant stud (211) is connected in a manner so as to be devoid of a guide sleeve, to its bottom terminal area with the wall of the longitudinal bore (13) of the primary cylinder (10) by means of an adhesive joint.

13. Endosseous implant according to any of the preceding Claims 1 thru 12, characterized in that the implant stud (211) is connected in a manner so as to be devoid of any guide sleeve by means of a screw connection (536) or some other suitable, equivalent connection to a shaped member (535) provided with an upper bore for accommodating the implant stud (211) retained within the interior of the primary cylinder (10) by means of an adhesive joint (537) filling the entire space otherwise receiving the guide tube inclusive of the cavity located therebelow between the bottom end of the otherwise provided guide tube (30) and the bottom of the primary cylinder.

14. Endosseous implant according to any of Claims 1 thru 13, characterized in that the primary cylinder (10) is fabricated from ceramic material, titanium or some other suitable materials.

15. Endosseous implant according to any of Claims 1 thru 14, characterized in that the implant stud (211) of the secondary cylinder (100) is retained in the primary cylinder (10) with the aid of a clamping closure (630) which comprises a shaped member (600) disposed within the longitudinal bore (13) of the primary cylinder (10) and provided with an upper mounting means (602) and a capsular element (603) disposed on the bottom end of the implant stud (211) which clampingly engages around the mounting means (602), said capsular element (603) being fabricated from a bi-metal, more particularly memorious metal.

16. Endosseous implant according to Claim 15, characterized in that the guide tube (30) mounted within the bottom region by means of an adhesive joint in the primary cylinder (10) is of short length and, within its interior, supports a shaped member (600) constructed in the form of a full cylinder, said member being fabricated from a metallic material or from some other suitable material, which, with the aid of an adhesive joint, is rigidly connected to the guide tube (30) and possesses a length which corresponds to that of the guide tube (30), and in that the shaped member (600), on its side (601) facing the modular element (220), bears a mounting means (602) of spherical or some other geometric configuration, around which a capsular element (603) engages, which, on the bottom end of the implant stud (211), is formed onto the same, and the wall area of which (603a) facing the mounting means (602) bears a bimetallic, e.g. memorious metal strip (604) or an internal coating, lining or the like of an elastic plastic or of some other suitable resilient material.

17. Endosseous implant according to Claim 16, characterized in that, within the bottom zone of the longitudinal bore (13), inside the same, a shaped member (600) is fitted with the aid of an adhesive joint, said member being provided at the top with a mounting means (602) possessing a spherical or some other suitable geometric configuration, around which a capsular element (603) engages which is secured to the bottom end of the implant stud (211) and is fabricated from a bimetal, more particularly from memorious metal.

18. Endosseous implant according to any of Claims 1 thru 17, characterized in that the mounting head (330) is attached with a clamping closure (630a) constructed in conformity with the clamping closure (630) on the implant stud (211) of the secondary cylinder (100), wherein the implant stud (211), on its upper end, is provided with a mounting means (602a) possessing a spherical or some other suitable geometric configuration, and in that the mounting head (330), within its interior, possesses a claw-like element (630a) which engages around the mounting means (602a) which is fabricated from a bimetal, more particularly from memorious metal.

19. Endosseous implant according to any of Claims 1 thru 18, characterized in that the implant stud (211) of the secondary cylinder (100) is retained in the primary cylinder (10) with the aid of a clamping closure (640) which comprises a shaped member (610) mounted within the longitudinal bore (13) of the primary cylinder (10), said member being provided with a central bore (611) fitted with an internal groove (612) and an annular bead (613) formed onto the outer circumference of the implant stud (211), said implant stud (211) being inserted into the primary cylinder (10) with said annular bead engaging or clicklocking into the annular groove (612).

20. Endosseous implant according to Claim 19, characterized in that the annular groove or grooves (612) of the bore (611) of the shaped member (610), for the purpose of constructing top undercuts (614), possesses or possess a cross-section which corresponds to a right-angled triangle, and in that the implant stud (211), at its bottom outer circumference, bears a number of annular engagement means (615), the number of which corresponds to that of the annular grooves (612) for the formation of a nondetachable closure.

21. Endosseous implant according to Claim 19, characterized in that the shaped member (610) is constructed with solid walls and, on its side facing the modular element (220), carries a guide tube (30), in that the shaped member (610) and the guide tube (30), with the aid of an adhesive joint, is rigidly connected to the inner wall area of the longitudinal bore (13), in that the shaped member (610) is dimensioned so as to be of approximately equal length as the guide tube (30), in that, between the guide tube (30) and the modular element (220), an air gap (225) is formed, in that the shaped member ((610) is provided with an upper bore (611) within the inner wall area (611a), of which at least one annular groove (612) is constructed, and in that the implant stud (211), at its lower end, is constructed in a conical fashion and, on its external circumferences, is provided with a number of annular beads (613) corresponding to the number of annular grooves (612) which can be click-locked into said annular grooves (612), in which the internal diameter of the bore (611) in the shaped member (610), in comparison with the external diameter of the implant stud (211), is dimensioned so as to be smaller and the external diameter of the implant stud corresponds to the internal diameter of the bore (611).

22. indosseous implant according to any of Claims 19 thru 21, characterized in that the mounting head (330) is secured by means of a clamping closure (640a) constructed in conformity with the clamping closure (640) for the implant stud (211) of the secondary cylinder (100), in which the implant stud (211), on the outer circumference of its upper free end, bears an annular bead (613a) which clicklocks into an annular groove (612a) in the inner wall area of a bore (611b) constructed in the mounting head (330).

23. Endosseous implant according to any of Claims 1 thru 22, characterized in that, for the formation of a gap sealing between two spherical gliding surfaces (212, 240c) of the primary cylinder (10) and the implant attachment section (240 ; 240a), at least one of the gliding surfaces is provided with a coating (620) or with a lining comprised of a polytetrafluoroethylene (R) or some other suitable material, more particularly a material pervious to oxygen ions.

24. Endosseous implant according to any of Claims 1 thru 23, characterized in that the air gap (225) between the guide tube (30) and the upper modular element (220) is filled by a tubular modular element (30a) of a permanently resilient material which is mounted above the guide tube (30).

25. Endosseous implant according to any of Claims 1 thru 24, characterized in that the upper circumferential rim of the modular element (220) is outwardly angled in a sleeve-like manner.

## FIG.1

*FIG.2*

*FIG.3*

FIG.4

FIG.5

*FIG.6*

*FIG.7*

*FIG.8*

FIG.9

FIG.10

FIG.11

FIG.12

_FIG.13_

212

α

225

10

B

A

13

30

215

_FIG.14_

315

240b

240a

220c

100

211

S

L

FIG.13

FIG.14

## FIG.17

## FIG.18

*FIG.22*

603a

330

*FIG.19*

602a

330

630a

603a

240

220

100

215

225

10

211

603

602

630

30

600

*FIG.20*

602a

220

240

100

211

603

*FIG.21*

100

215

13

602

600

FIG.23

FIG.26

FIG.25

FIG.24

FIG.27

315

240a

220

240c

225

10

612

613

30

610

614

615

A

B

611

FIG.28

315

240a

240

240c

225

10

240b

212

612

211

100

30a

30

215

240b

212

211

100

215